(19) Europäisches Patentamt
European Patent Office
Office européen des brevets

(11) EP 4 286 369 A1

(12) EUROPEAN PATENT APPLICATION

(43) Date of publication:
06.12.2023 Bulletin 2023/49

(21) Application number: 22305816.5

(22) Date of filing: 03.06.2022

(51) International Patent Classification (IPC):
C07C 255/23 (2006.01) C09J 4/00 (2006.01)
C07D 307/00 (2006.01)

(52) Cooperative Patent Classification (CPC):
C07C 255/23; C07D 307/12

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Bostik SA
92700 Colombes (FR)

(72) Inventors:
- FAGGI, Enrico
08193 BELLATERRA, BARCELONA (ES)
- COURT, François
92700 FR (FR)
- GARRA, Patxi
08193 BELLATERRA, BARCELONA (ES)
- ESCRIBA PLA,, Marc
08193 BELLATERRA, BARCELONA (ES)

(74) Representative: Arkema Patent
Arkema France
DRD-DPI
420, rue d'Estienne d'Orves
92705 Colombes Cedex (FR)

# (54) BIOBASED CYANOACRYLATE COMPOUND

(57) The present invention concerns a compound of formula (I):

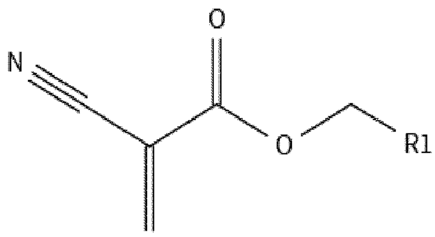

(I)

wherein R1 is H or an organic moiety, said compound of formula (I) having a biocarbon content equal to or higher than 20%.

EP 4 286 369 A1

## Description

Field of the invention

The present invention relates to the field of biobased cyanoacrylate.

The present invention also relates to the use of said biobased cyanoacrylate for preparing adhesive composition, and to the resulting adhesive compositions.

Background art

Cyanoacrylates (CAs) is the generic name for a family of resistant fast acting adhesives based on esters of 2-cyanoacrylic acid. Such compounds have been well known for some time, as described in, for example, S. Ebnesajjad Ed., Adhesives Technology Handbook, William Andrew, Norwich, 2008.

Adhesive compositions based on cyanoacrylate (CA) esters are also well-known, e.g. as instant adhesives or so-called 'superglues'. They are popular in many areas of application and are used by consumers, professional craft workers and industrial assemblers. They are typically solvent free, 100% reactive materials, noted for their ability to form strong adhesive bonds on many different substrates in seconds.

In many industrial and domestic applications these compounds are used in form of one-component or two components as they polymerize rapidly when they form a thin film between two substrates in the presence of anions or nucleophilic species. The speed at which the bond is formed and the ease of use have contributed to their popularity.

While efforts are made to improve their properties (toughening, temperature, humidity...) the synthetic path for the cyanoacrylate itself remains mainly the same industrially since the end of the 1950ies. Conventional cyanoacrylates are derived from petrochemical-based chemicals, and rely on energy-intensive processes. Use of these materials contributes to the increase in the greenhouse effect. With decreasing world petroleum reserves, the sources of these raw materials are gradually being exhausted. This is an emergency especially in regard to 2050 carbon neutral objectives of many countries.

There is an increased desire to reduce carbon footprint and to produce products which are less toxic, more environment-friendly.

Raw materials derived from biomass are from a renewable source and have a reduced impact on the environment. They do not require all the energy-consuming refining stages of petroleum products. Production of $CO_2$ is reduced, so that they contribute less to global warming.

However, such sustainability developments should not impact negatively the current properties of cyanoacrylate, such as for example the fixturing time, adhesive properties, etc..

There is thus a need for providing environment-friendly cyanoacrylates that allows to resolve at least a part of the above-mentioned drawbacks.

More particularly, there is a need for more environment-friendly cyanoacrylates which exhibit fast fixturing time.

More particularly, there is a need for more environment-friendly cyanoacrylates which exhibit at least similar or better properties than conventional petroleum-based cyanoacrylates.

Besides, there is a need for more environment-friendly cyanoacrylate which can be produced industrially with good yields.

Description of the invention

**COMPOUND**

The present invention concerns a compound of formula (I):

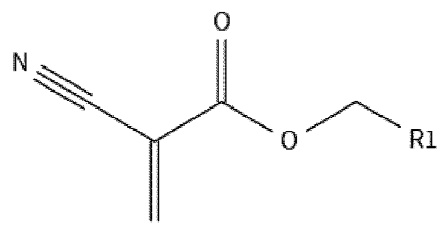

(I)

wherein R1 is H or an organic moiety, said compound of formula (I) having a biocarbon content equal to or higher than 20%.

The carbon of a biomaterial derives from the photosynthesis of plants and therefore from atmospheric $CO_2$. The degradation (by degradation is also meant combustion/incineration at the end of their life) of these materials into $CO_2$ does not therefore contribute to warming since there is no increase in the carbon emitted into the atmosphere. The $CO_2$ balance of biomaterials is therefore clearly improved and contributes to reducing the carbon footprint of the products obtained (only the energy for manufacturing is to be taken into account). On the contrary, a material of fossil origin which also degrades into $CO_2$ will contribute to an increase in the level of $CO_2$ and therefore to global warming.

The components of the invention will therefore have a carbon footprint which will be better than that of compounds obtained from a fossil source.

The terms "biocarbon" or "biobased carbon" indicates that the carbon is of renewable origin, or of natural origin and originates from a biomaterial, as indicated below. The biocarbon content and the biomaterial content are expressions denoting the same value.

A material of renewable origin, also called biomaterial, is an organic material in which the carbon derives from $CO_2$ recently fixed (on a human scale) by photosynthesis from the atmosphere. On earth, this $CO_2$ is captured or fixed by plants. At sea, $CO_2$ is captured or fixed by bacteria or plankton carrying out photosynthesis. A biomaterial (100% carbon of natural origin) has a $^{14}C/^{12}C$ isotope ratio greater than $10^{-12}$, typically of approximately 1.2 x $10^{-12}$, while a fossil material has a zero ratio. Indeed, the isotope $^{14}C$ is formed in the atmosphere and is then integrated by photosynthesis, according to a time scale of a few decades at most. The half-life of $^{14}C$ is 5730 years. Thus, materials resulting from photosynthesis, namely plants in general, necessarily have a maximum $^{14}C$ isotope content.

The biomaterial content or biocarbon content is determined by using the standards ASTM D 6866 (ASTM D 6866-21, method B) and ASTM D 7026 (ASTM D 7026-04). The ASTM D 6866 standard is "Determining the Biobased Content of Natural Range Materials Using Radiocarbon and Isotope Ratio Mass Spectrometry Analysis", while the ASTM D 7026 standard is "Sampling and Reporting of Results for Determination of Biobased Content of Materials via Carbon Isotope Analysis". The second standard makes reference in its first paragraph to the first standard.

The first standard describes a test for measuring the $^{14}C/^{12}C$ ratio of a sample and compares it with the $^{14}C/^{12}C$ ratio of a reference sample of 100 % renewable origin, to give a relative percentage of C of renewable origin in sample. The standard is based on the same concepts as $^{14}C$ dating, but without applying the dating equations.

The ratio thus calculated is referred to as the "pMC" (percent Modern Carbon). If the material to be analyzed is a mixture of biomaterial and fossil material (without radioactive isotope), then the pMC value obtained is directly correlated with the quantity of biomaterial present in the sample.

A C-14 modern standard (Oxalic Acid II) represents what modern (living plant) C-14 activity was in 1950 which has been set as the standard for 100% biobased activity. A background level is also established which is subtracted from the activity measured. This activity of the sample is compared to the activity of the Oxalic Acid II. This will give the pMC or percent of modern carbon. If there is a mixture of modern carbon and fossil carbon in the sample, then the resulting pMC (example 85 pMC) is indicating that we measured 85 percent of the activity of the modern standard. The sample would be reported as 85% biobased carbon. Due to nuclear tests in the atmosphere in the 1950's and 1960's C-14 was artificially created in the atmosphere. At one point it was nearly 200% of natural activity. Over time, with the dilution of fossil fuel $CO_2$ being put into the atmosphere this activity has been diluted such that today the C-14 activity in the atmosphere is back to the same level as the modern standard. There is an atmospheric correction factor that is used to account for these changes in the atmospheric C-14 activity. This atmospheric correction factor (REF) was used as following:

$$\text{Biocarbon content} = \text{pMC} / (\text{REF})$$

with REF = 1.000 (in 2022)

The precision on the biocarbon content % is of +/- 3% (absolute).

The compound of formula (I) as defined herein preferably has a biocarbon content equal to or higher than 25%, more preferably equal to or higher than 41%, even more preferably equal to or higher than 50%, and more advantageously equal to or higher than 55%.

In a preferred embodiment, R1 is H or a hydrocarbonated radical which may comprise at least one heteroatom.

Preferably, R1 is selected from the group consisting of: H, alkyl, halogenated alkyl, alkyl silane, acetoxy silane, alkenyl, alkynyl, aryl, heteroaryl, alkaryl, aralkyl, cycloalkyl, cycloalkenyl, heterocycloalkyl, alkoxyalkyl, acrylic ester moiety, oxetane moiety, epoxy moiety, carboxylic ester moiety.

The alkyl, alkenyl, aryl, alkynyl, heteroaryl, alkaryl, aralkyl, cycloalkyl, cycloalkenyl, heterocycloalkyl, alkoxyalkyl may be substituted, for example by at least one substituent selected from the group consisting of: alkyl, aryl, alkenyl, OH, amine, amide, halogen atom, - $CF_3$, and mixtures thereof.

As used herein, the term « alkyl » means a linear or branched hydrocarbon radical Examples are methyl, ethyl, propyl.

As used herein, the term « alkenyl » means a linear or branched hydrocarbon radical comprising at least one double bond. Examples are allyl, propenyl, butenyl.

As used herein, the term "alkynyl" means a linear or branched hydrocarbon radical comprising at least one triple bond. Example is propargyl.

As used herein, the term « aryl » means a monocyclic or bicyclic aromatic radical, preferably comprising from 6 to 12 carbon atoms. Phenyl is an example.

As used herein, the term « heteroaryl » means an monocyclic or bicyclic aromatic radical comprising at least one heteroatom such as for example O, S or N, and preferably comprising from 4 to 12 carbon atoms. Some examples are furanyl, thiophenyl, pyrrolyl, pyridinyl, indolyl or imidazolyl.

As used herein, the term « arylalkyl » or "aralkyl", means an alkyl group substituted by an aryl group, said arylalkyl preferably comprising from 7 to 20 carbon atoms. One example is benzyl group.

As used herein, the term « alkylaryl » or « alkaryl" means an aryl group substituted by an alkyl group, said alkylaryl preferably comprising from 7 to 20 carbon atoms.

As used herein, the term « cycloalkyl » means a monocyclic or polycyclic, saturated system, preferably comprising from 3 to 12 carbon atoms. Examples of cycloalkyl groups are cyclopropyl, cyclopentyl, cyclohexyl, cycloheptyl, or norbornyl.

As used herein, the term « cycloalkenyl » means a monocyclic or polycyclic, unsaturated system, preferably comprising from 3 to 12 carbon atoms.

As used herein, the term « heterocycloalkyl » means monocyclic or polycyclic, saturated system comprising at least one heteroatom such as for example O, S or N, preferably comprising from 3 to 12 carbon atoms. Example is tetrahydrofurfuryl or tetrahydrothiophene groups.

As used herein, the term « alkoxyalkyl » means a radical -alkyl-O-alkyl, with the alkyl being as defined above.

When R1 is an alkyl group, it may be selected from methyl, ethyl, n-propyl, iso-propyl, n-butyl, sec-butyl, isobutyl, tert-butyl, n-pentyl, 1-methylbutyl, 1-ethylpropyl, neopentyl, n-hexyl, 2-heptyl, 1-methylpentyl, n-heptyl, n-octyl, 2-octyl, 2-ethylhexyl, n-nonyl, n-decyl, n-undecyl.

When R1 is alkyl silane, it may be selected from ethyltrimethyl silane, methyltrimethylsilane or propyltrimethylsilane.

When R1 is an alkenyl group, it may be selected from allyl, propenyl, butenyl.

When R1 is an alkynyl group, it may be propargyl.

When R1 is an aryl group, it may be selected from phenyl, methylphenyl.

When R1 is a heteroaryl group, it may be selected from furanyl, methylfuranyl, thiophenyl, pyrrolyl, pyridinyl, indolyl, imidazolyl.

When R1 is arylalkyl, it may be benzyl.

When R1 is a cycloalkyl group, it may be selected from cyclopropyl, cyclopentyl, cyclohexyl, cycloheptyl, isobornyl, methylbornyl, or norbornyl.

When R1 is a cycloalkenyl group, it may be selected from cyclohexenyl, cycloheptenyl.

When R1 is a heterocycloalkyl group, it may be selected from methyltetrahydrofuryl, tetrahydrofuryl.

When R1 is an alkoxyalkyl group, it may be selected from 2-methoxymethyl, 2-ethoxymethyl, 2-propoxymethyl, 2-butoxymethyl, 2-isopropoxymethyl, 2-hexyloxymethyl, 2-amyloxymethyl, 2-ethoxypropyl, 2-methoxyethyl, and 2-(1-methoxy)ethyl. Other examples of alkoxyalkyl groups are described in US6977278.

When R1 is an acrylic ester moiety, it preferably has the following formula:

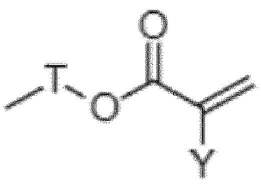

wherein T is selected from $(CH_2)_z$, z being comprised from 1 to 12, preferably from 1 to 6 ; a branched C2-C12 alkylene ; alkynylene ; alkenylene ; alkylene-O-alkylene ; cyclohexylene, biphenylene ; $-C_6H_4C(Me)_2C_6H_4-$ ; $-C_6H_4CH_2C_6H_4-$ ; phenylene; $-C_6H_4$-alkylene- ;
and Y is H, Me or CN

When R1 is a carboxylic ester moiety, it preferably has the formula $-(CH_2)_kCO_2Y'$ or - $C(=CH_2)-CO_2Y'$, with k being comprised from 1 to 18, preferably from 1 to 6, and Y" being a C1-C4 alkyl group.

The compound of formula (I) may have one of the following formulae (I-A), (I-B), or (I-C)

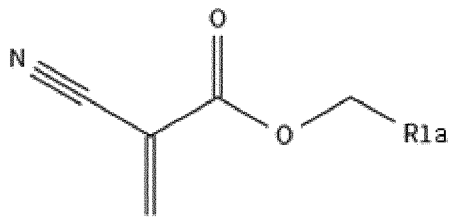

(I-A)

wherein R1a is alkyl,

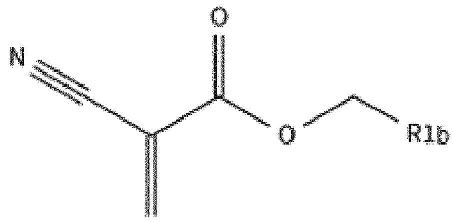

(I-B)

wherein R1b is selected from H, alkyl silane, acetoxy silane, alkenyl, alkynyl, aryl, heteroaryl, alkaryl, aralkyl, cycloalkyl, cycloalkenyl, heterocycloalkyl, alkoxyalkyl, oxetane moiety, epoxy moiety, preferably R1b being selected from cycloalkyl, aralkyl, heteroaryl, heterocycloalkyl, alkoxyalkyl;

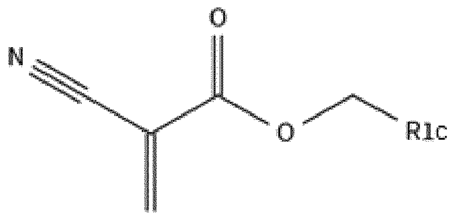

(I-C)

wherein R1c selected from acrylic ester moiety and carboxylic ester moiety.

Examples of compound of formula (I-A) may be ethylcyanoacrylate, n-propylcyanoacrylate, n-butylcyanoacrylate, isobutylcyanoacrylate, n-heptylcyanoacrylate, n-octylcyanoacrylate, n-hexylcyanoacrylate, n-pentylcyanoacrylate, nonylcyanoacrylate, dodecyanoacrylate. Preferred compounds of formula (I-A) are ethylcyanoacrylate, n-heptylcyanoacrylate.

Examples of compound of formula (I-B) may be: methylcyanoacrylate, tetrahydrofurfuryl cyanoacrylate, 2-methoxyethylcyanoacrylate, 2-ethoxyethylcyanoacrylate, 2-methoxybutylcyanoacrylate, 2-propoxyethylcyanoacrylate, propargylcyanoacrylate, 2-phenylethylcyanoacrylate, cyclohexenylcyanoacrylate, trialkylsilylethylcyanoacrylate, trialkylsilylpropylcyanoacrylate, trialkylsilylbutylcyanoacrylate, benzylcyanoacrylate.

Examples of compound of formula (I-C) may be:

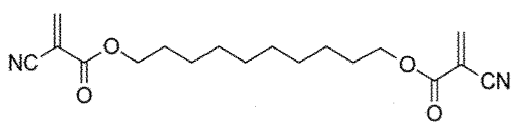

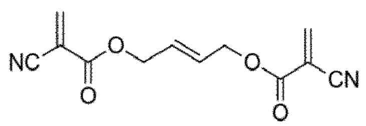

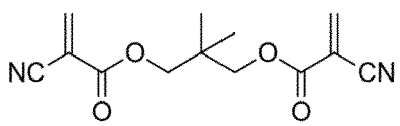

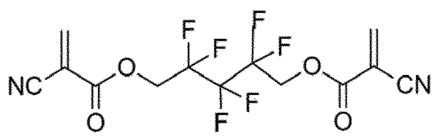

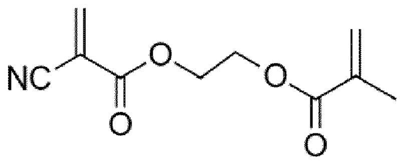

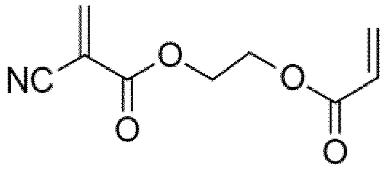

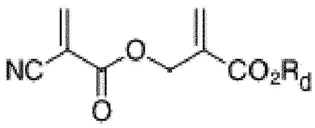

wherein Rd is methyl or ethyl

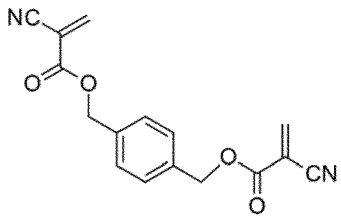

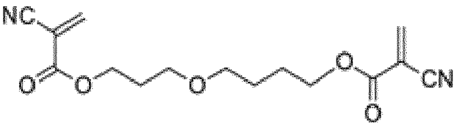

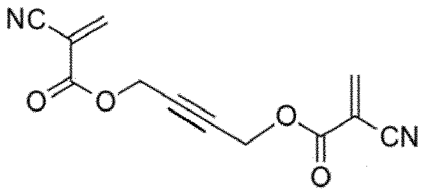

Preferably, the compound of formula (I) is a compound of formula (I-A) or (I-B).

The compound of formula (I) may be selected from the group consisting of methylcyanoacrylate, ethylcyanoacrylate, n-propylcyanoacrylate, n-butylcyanoacrylate, isobutylcyanoacrylate, n-heptylcyanoacrylate, n-octylcyanoacrylate, n-hexylcyanoacrylate, n-pentylcyanoacrylate, nonylcyanoacrylate, dodecyanoacrylate, tetrahydrofurfuryl cyanoacrylate, 2-ethoxyethylcyanoacrylate, 2-propoxyethylcyanoacrylate, 2-methoxyethylcyanoacrylate, 2-methoxybutylcyanoacrylate, trialkylsilylethylcyanoacrylate, trialkylsilylpropylcyanoacrylate, trialkylsilylbutylcyanoacrylate, benzylcyanoacrylate, propargylcyanoacrylate, 2-phenylethylcyanoacrylate, cyclohexenylcyanoacrylate.

Preferably, in formula (I), R1 is selected from alkyl group and heterocycloalkyl.

Preferably, in formula (I), R1 is a C1-C20 alkyl group, more preferably C1-C10 alkyl group, even more preferably C6 alkyl group.

Preferably, in formula (I) herein, when R1 is methyl, the biocarbon content of the compound of formula (I) is higher or equal to 41%, more preferably higher or equal to 50%.

## PROCESS

The present invention also concerns a process of preparation of a compound of formula (I):

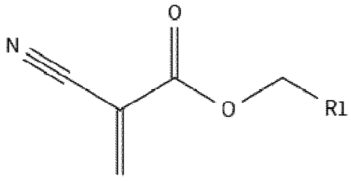

(I)

wherein R1 is H or an organic moiety, said compound of formula (I) having a biocarbon content equal to or higher than 20%.

All the embodiments and preferred features disclosed for the compound of formula (I) above (in paragraph

"COMPOUND") apply to the its process of preparation and are not reiterated.

The process of preparation of a compound of formula (I) as defined herein, preferably comprises a step of converting a cyanoacetate of formula (II) into a compound of formula (I) as defined herein:

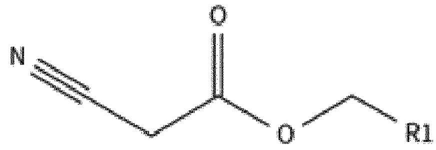

(II)

wherein R1 in formula (II) is as defined in formula (I) herein, namely R1 is H or an organic moiety.

All the embodiments and preferred features disclosed for R1 in the compound of formula (I) apply herein for R1 in the compounds of formula (II), and are not reiterated.

The compound of formula (II) may have one of the following formulae (II-A), (II-B), or (II-C) :

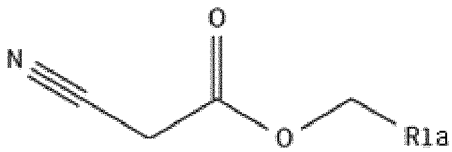

(II-A)

wherein R1a is alkyl;

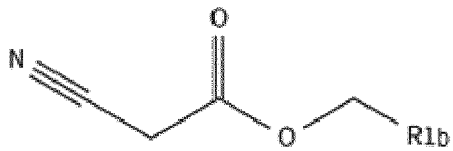

(II-B)

wherein R1b is selected from H, alkyl silane, acetoxy silane, alkenyl, alkynyl, aryl, heteroaryl, alkaryl, aralkyl, cycloalkyl, cycloalkenyl, heterocycloalkyl, alkoxyalkyl, oxetane moiety, epoxy moiety; preferably R1b being selected from cycloalkyl, aralkyl, heteroaryl, heterocycloalkyl, alkoxyalkyl;

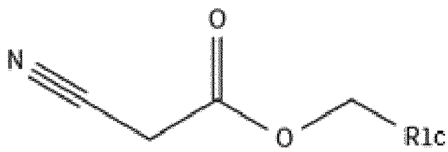

(II-C)

wherein R1c is selected from acrylic ester moiety and carboxylic ester moiety.

Examples of compound of formula (II-A) may be ethylcyanoacetate, n-propylcyanoacetate, n-butylcyanoacetate, isobutylcyanoacetate, n-heptylcyanoacetate, n-octylcyanoacetate, n-hexylcyanoacetate, n-pentylcyanoacetate, nonylcyanoacetate, dodecyanoacetate. Preferred compounds of formula (II-A) are ethylcyanoacetate, n-heptylcyanoacetate.

Examples of compound of formula (II-B) may be: methylcyanoacetate, tetrahydrofurfuryl cyanoacetate, 2-ethoxyethylcyanoacetate,2-methoxyethylcyanoacetate, 2-methoxybutylcyanoacetate, 2-propoxyethylcyanoacetate, propargylcyanoacetate, 2-phenylethylcyanoacetate, cyclohexenylcyanoacetate, trialkylsilylethylcyanoacetate, trialkylsilylpropylcyanoacetate, trialkylsilylbutylcyanoacetate, benzylcyanoacetate.

Examples of compound of formula (II-C) may be

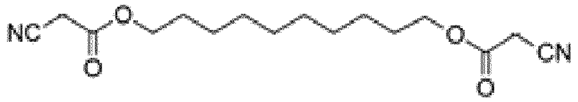

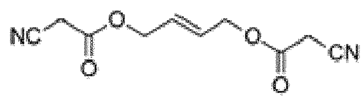

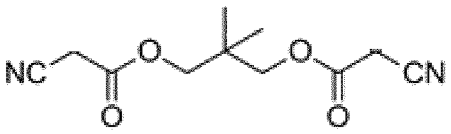

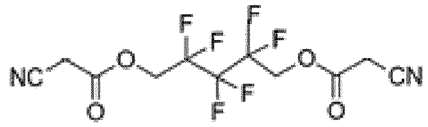

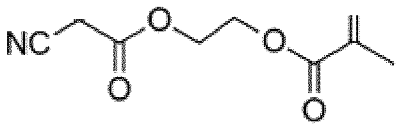

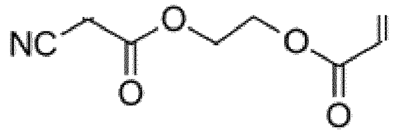

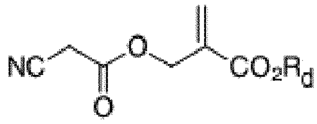

wherein Rd is methyl or ethyl

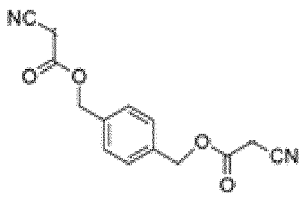

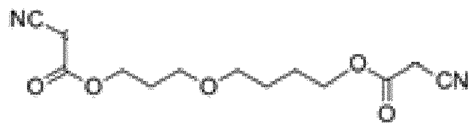

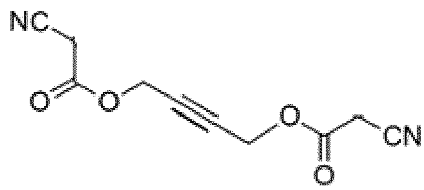

Preferably, the compound of formula (II) is a compound of formula (II-A) or (II-B).

Preferably, in formula (II), R1 is selected from alkyl and heterocycloalkyl.

Preferably, in formula (II), R1 is a C1-C20 alkyl group, more preferably C1-C10 alkyl group, even more preferably C6 alkyl group.

Preferably, in formula (II) herein, when R1 is methyl, the biocarbon content of the compound of formula (II) is higher or equal to 25%, more preferably higher or equal to 50%.

The compound of formula (II) preferably has a biocarbon content equal to or higher than 25%, more preferably equal to or higher than 45%, and even more preferably equal to or higher than 50%.

The present invention also concerns a compound of formula (I) as defined herein susceptible to be obtained by a process comprising a step of converting a cyanoacetate of formula (II) as defined herein into a compound of formula (I).

The process of preparation of a compound of formula (I):

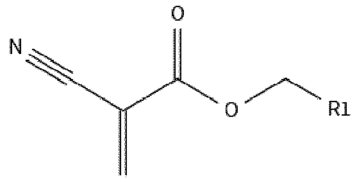

(I)

wherein R1 is H or an organic moiety, said compound of formula (I) having a biocarbon content equal to or higher than 20%, is preferably selected from process P1 or process P2 as defined hereafter.

**Process P1**

The present invention concerns a process of preparation P1 of a compound of formula (I) as defined above which comprises:

- a step S1 comprising the reaction of condensation of a compound of formula (II) :

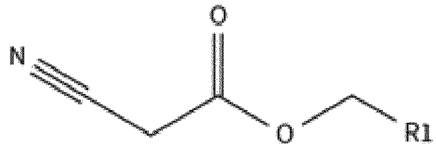

(II)

as defined above, with a formaldehyde derivative; then
- an optional step S2 comprising the washing of the product obtained in step S1;
- a step S3 comprising the depolymerization of the product obtained at step S1 or S2 to provide a compound of formula (I),
- an optional step S4 of purification of the compound of formula (I) obtained at the end of step S3.

Step S1 is typically a Knoevenagel condensation reaction.
The step S1 is preferably carried out in presence of a basic catalyst, which may be selected from the group consisting of: amine, sodium hydroxide, potassium hydroxide, alkali metal alkoxide, and mixture thereof. The amine catalyst may be piperidine, ethanolamine or diethylamine.
The content of basic catalyst may range from 0.01 to 10 mol% based on the compound of formula (II).
The molar ratio of compound of formula (II) : formaldehyde derivative may range from 1 : 0.5 to 1 : 1.5.
The step S1 may be carried out in a solvent which may be selected from the group consisting of: benzene, toluene, chloroform, trichloroethylene, tetrahydrofuran, water, and mixture thereof.
The step S1 is preferably carried out at a temperature ranging from 30°C to 150°C, more preferably from 50°C to 100°C.
The optional step S2 comprising the washing may be carried out with water or an aqueous acidic solution. The aqueous acidic solution may be prepared with an acid selected from the group consisting of sulfuric acid, hydrochloric acid, phosphoric acid, formic acid, acetic acid, and mixture thereof. The concentration of the acid preferably ranges from 0.01 to 5 % by weight in the aqueous acidic solution.
The washing may be carried out at a temperature ranging from 0°C to 100°C.
The organic phase may be recovered by decantation, and the solvent may be evaporated under reduced pressure.
The step S3 comprising the depolymerization of the product obtained at step S1 or S2 may be carried out at a temperature ranging from 100°C to 250°C, preferably from 140°C to 200°C, preferably under reduced pressure.
The step S3 is preferably implemented in the presence of a depolymerization catalyst such as phosphorus pentoxide, phosphoric acid, or polyphosphoric acid.
The purification step S4 may be a distillation, double distillation, crystallization.
The formaldehyde derivatives typically include paraformaldehyde and substances that produces formaldehyde in the reaction (precursors of formaldehyde). Preferably, the formaldehyde derivative is paraformaldehyde.
Examples of substances producing formaldehyde in the reaction are paraformaldehyde or the like.
The formaldehyde derivative may derived from petroleum-based material, or may be partially biobased or even fully biobased (biocarbon content of 100%).

Biobased formaldehyde may be produced from conventional partial methanol oxidation (« Formaldehyde », Ullmann's Encyclopedia of Industrial Chemistry, Weinheim, Wiley-VCH, 2000), using biomethanol (Veolia).

As used herein, the term "partially biobased" means that the biocarbon content of the material is at least higher than 0%, preferably at least higher than 10%.

Preferably, the process P1 comprises:

- a step S1;
- a step S2 comprising the washing of the product obtained in step S1;
- a step S3 comprising the depolymerization of the product obtained at step S1 or S2 to provide a compound of formula (I),
- a step S4 of purification of the compound of formula (I) obtained at the end of step S3.

The embodiments and preferred embodiments for compounds of formula (II) disclosed above apply in the process P1 and are not reiterated.

**Process P2**

The present invention concerns a process of preparation P2 of a compound of formula (I) as defined above which comprises:

- a step comprising the reaction of compound of formula (II)

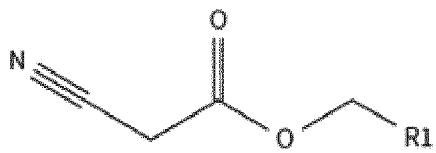
(II)

as defined above, with a compound of formula (X):

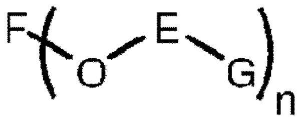
(X)

wherein E is $(CH_2X)_m$, m is comprised between 1 and 20, X is O or S,
when n=1, F is selected from the functional groups:

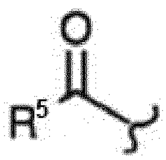 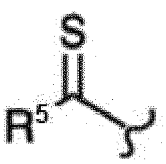 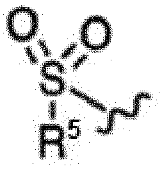 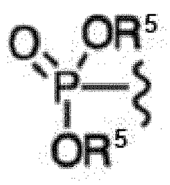

when n=2, F is selected from the functional groups:

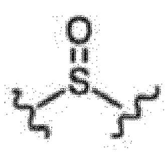 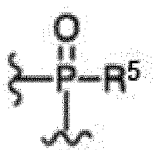 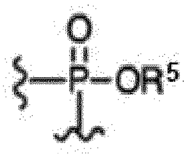

when n= 3, F is:

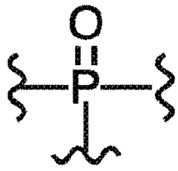
,

G is selected from the functional groups:

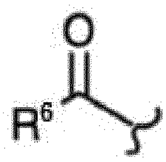 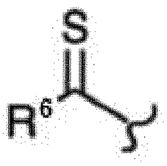 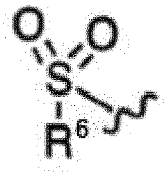 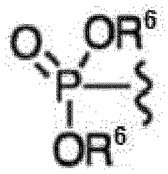

G and F are independently selected from each other, and
R5 and R6 are independently of each other selected from the functional groups H, linear or branched C1-C4 alkyl, C1-C4 halogenated alkyl, carboxy substituted C1-C4 alky, C3-C10 cycloalkyl, aryl, and heterocycloalkyl, or when n = 1, F may be connected to G through one R7 group selected from $(CH_2)_v$, $(CHR7)_v$, $(CR7R8)_v$, where v ranges from 1 to 6, preferably from 1 to 4, and more preferably v is 1, R7 and R8 being the same or different C1-C4 alkyl groups,
in presence of a catalytic amount of an ammonium or iminium salt (XI);

- an optional step of isolation of the compound obtained (II) obtained.

This process is typically disclosed in WO2015/150882.
The ammonium or iminium salt (XI) may be in homogeneous phase or supported on a solid substrate, preferably in homogeneous phase.
As used herein, the term "ammonium salt" means the product resulting from the reaction for example of an acid with a primary amine (for example methylamine, ethylamine, ethylenediamine, aniline, benzylamine), a secondary amine (for example piperazine) or tertiary amine (for example N,N'-dimethoxymethylpiperazine).
As used herein, the term "iminium salt" means the imine resulting from the reaction of a primary or secondary amine with an aldehyde or with a ketone, in neutral or acidic medium, which leads to an iminium cation, and that contains an anion from an acid.
The acid employed for the preparation of the ammonium salt and the iminium salt can be any acid. Preferably the acid is selected from the group consisting of acetic acid, trifluoroacetic acid, sulfuric acid, methanesulfonic acid, p-toluenesulfonic acid, dodecylbenzenesulfonic acid, camphorsulfonic acid, hydrochloric acid, phosphoric acid, and mixtures thereof; preferably the acid is selected from the group consisting of sulfuric acid, methanesulfonic acid, p-toluenesulfonic acid, dodecylbenzenesulfonic acid, and mixtures thereof, and more preferably from methanesulfonic acid, p-toluenesulfonic acid, dodecylbenzenesulfonic acid, and mixtures thereof. It is also possible to employ mixtures of acids and/or mixtures of amines.
The ammonium salt and the iminium salt (XI) may be formed in situ during the execution of the process of the invention, or alternatively can be prepared separately and added independently to the reaction mixture. Preferably, they are prepared in situ.
Preferably E is $(CH_2X)_m$, m is comprised between 1 and 10, X is O or S, n= 1 , F is selected from the functional groups:

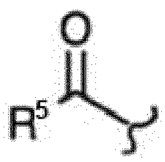 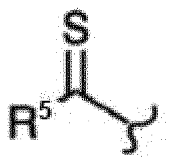 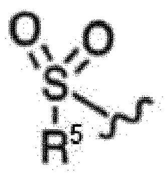 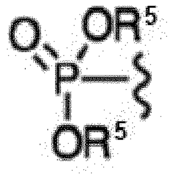

G is selected from the functional groups:

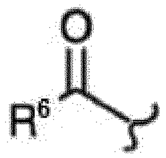 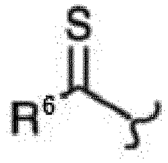 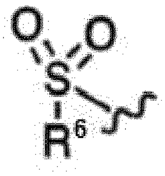 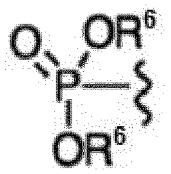

G and F are independently selected from each other, and

R5 and R6 are independently of each other selected from the functional groups H, linear or branched C1-C4 alkyl, C1-C4 halogenated alkyl, carboxy substituted C1-C4 alkyl, C3-C10 cycloalkyl, aryl, and heterocycloalkyl, or

when n = 1, F may be connected to G through a R7 group selected from $(CH_2)_v$, $(CHR7)_v$, $(CR7R8)_v$, where v ranges from 1 to 6, preferably from 1 to 4, and more preferably v is 1, R7 and R8 being the same or different C1-C4 alkyl groups,

More preferably, E is $(CH_2X)_m$, m is comprised between 1 and 3, X 1 , F is:

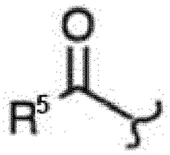

G is:

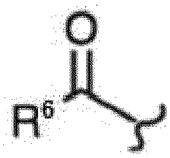

G and F are independently selected from each other, and

R5 and R6 are independently of each other selected from the functional groups H, linear or branched C1-C4 alkyl, C1-C4 halogenated alkyl, carboxy substituted C1-C4 alky, C3-C10 cycloalkyl, aryl, and heterocycloalkyl, preferably R5 and R6 are independently selected from each other from the functional groups H and linear or branched C1-C4 alkyl.

This type of compound can be obtained, for example, by reaction of formaldehyde gas or a source of formaldehyde, such as paraformaldehyde or trioxane, or a source of thioformaldehyde such as trithiane, with acid anhydrides such as, for example, acetic anhydride, thioacetic anhydride, dithiocarboxylic anhydride, methanesulfonic acid anhydride, succinic anhydride, phthalic anhydride, or tert- butoxycarboxylic acid anhydride.

The source of formaldehyde may be biobased (as disclosed herein in process P1 above).

When X is O (oxygen), and n is 1, the compounds are referred to as methylene carboxylic esters, methylene thiocarboxylic esters, methylene sulfonic esters or methylene phosphoric esters, depending on the groups F and G according to the above definitions. The preparation of methylene carboxylic esters is described, for example, in the US3927078.

When X is O (oxygen) and n is 2, the compounds are referred to as oxybismethylene carboxylic esters, oxybismethylene thiocarboxylic esters, oxybismethylene sulfonic esters, or oxybismethylene phosphoric esters, depending on the groups F and G according to the above definitions. The preparation of polyoxymethylene polycarboxylates is described, for example, in the US3219630. The preparation of oxybismethylene carboxylic esters is described, for example, in US3927078 and US3931412. The preparation of oxybismethylene sulfonic esters is described, for example, in the US4100200.

The preparation of trioxymethylene carboxylic esters (X is O and n = 3) is described, for example, in US3931412. The preparation of a tris(acyloxymethylene) phosphoric ester is described in the international patent application WO-A-96/40695.

When X is S (sulfur) and n is 1 , the compounds are referred to as methylene sulfide carboxylic esters, methylene sulfide thiocarboxylic esters, methylene sulfide sulfonic esters or methylene sulfide phosphoric esters, depending on the groups F and G according to the above definitions.

Among the compounds of general formula (X), the ones listed in the following table may be preferably mentioned:

| Name | Structure |
|---|---|
| Methylene diacetate | 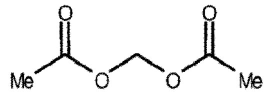 |
| Oxybismethylene diacetate | 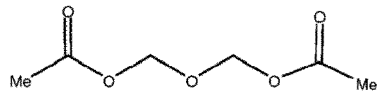 |
| Methylene dipropionate | 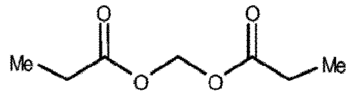 |
| Oxybismethylene dipropionate | 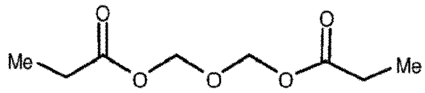 |
| Oxybismethylene benzoate propionate | 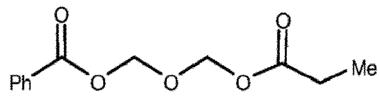 |
| Oxybismethylene dimesylate | 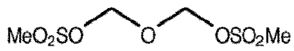 |
| (Methylsulfonyloxy)methyl-3-methylbutanoate | 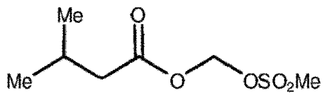 |

| | |
|---|---|
| Methylene methanedisulfonate | 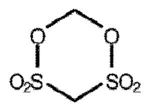 |
| Tris(acetoxymethylene) phosphate | 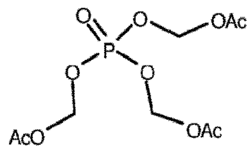 |

As compounds of formula (X) the preferred compounds are methylene diacetate, oxybismethylene diacetate, methylene dipropionate, and oxybismethylene dipropionate, more preferred are the compounds methylene diacetate and oxybismethylene diacetate, and still more preferred is methylene diacetate.
Preferably, the compound of formula (I) of the invention is obtained from process P2.
The compounds of formula (II) as defined herein may be obtained by one of the following processes P-A, P-B, P-C, P-D or P-E as described below.

*Process P-A*

The process P-A of preparation of a compound of formula (II) may comprise:

- contacting a compound of formula (III) with a compound of formula (IV), in the presence of an acid, more preferably under conditions sufficient to yield a compound of formula (II):

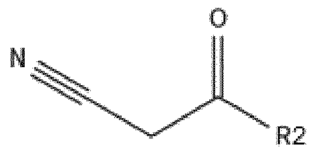 (III) 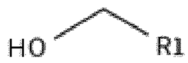 (IV)

wherein R1 is as defined above for formula (I), namely H or an organic moiety,
wherein R2= NRR' with each of R and R' being, independently of each other H, or an alkyl group;

- optionally separating therefrom the compound of formula (II).

The acid may be any acid catalyst known to the man skilled in the art. They can be either Lewis or Bronsted acids. The acid may be selected from the group consisting of Sulfuric Acid, p-toluene Sulfonic Acid, Scandium Triflate, Methanesulfonic Acid, Dodecylbenzene Sulfonic Acid, Boron Trifluoride, Phosphoric Acid, Perchloric Acid, Hydrochloric Acid. Also heterogeneous catalysts may be used, for example: Zirconium Tetrasulfate, Amberlyst 46, Acid-modified Montmorillonite (K10) clay, and Tungstated zirconia.
Preferably, the acid is a mineral acid, even more preferably selected from the group consisting of sulfuric acid, sulfurous acid, sulfonic acid, phosphoric acid, phosphorous acid, phosphonic acid, hydrochloric acid or hydrobromic acid.
The compound of formula (IV) should be used in excess to either or both the compound of formula (III) and the mineral acid.
The compound of formula (IV) may be selected from:

- compound of formula (IV-A) :

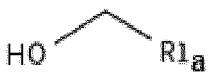

wherein R1a is alkyl;

- compound of formula (IV-B) :

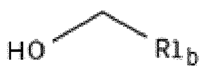

wherein R1b is selected from H, alkyl silane, acetoxy silane, alkenyl, alkynyl, aryl, heteroaryl, alkaryl, aralkyl, cycloalkyl, cycloalkenyl, heterocycloalkyl, alkoxyalkyl, oxetane moiety, epoxy moiety; preferably R1b being selected from cycloalkyl, aralkyl, heteroaryl, heterocycloalkyl, alkoxyalkyl;

- compound of formula (IV-C):

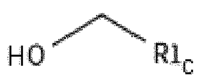

wherein R1c is selected from acrylic ester moiety and carboxylic ester moiety.

Preferably, in formula (IV), R1 is selected from alkyl group and heterocycloalkyl.
Preferably, in formula (IV), R1 is a C1-C20 alkyl group, more preferably C1-C10 alkyl group, even more preferably C6 alkyl group.
The compound of formula (IV) may be partially biobased, preferably fully biobased. In that case, it may be obtained from fermentation, sugars, pyrolysis of oils, organic solutions (juices), bio-oils...
The compound of formula (IV) preferably has a biocarbon content equal to or higher than 20%, more preferably equal to or higher than 50%, even more preferably equal to or higher than 80%, and more advantageously equal to or higher than 99%.
The compound of formula (IV) is typically commercialized by different company.
Examples of at least partially biobased or fully biobased compounds of formula (IV) are biosourced EtOH supplied by Myhome aromas, biosourced heptanol supplied by Arkema Oleris, Viridisol® T Tetrahydrofurfuryl Alcohol (THFA) supplied by Pennakem), isobutanol (supplied by Gevo), biomethanol (Veolia), n-Butanol supplied by Green

biologics, n-propanol supplied by Braskem, 1,10 dodecandiol supplied by Sartomer.

The compound of formula (IV) may be selected from the group consisting of methanol, ethanol, n-propanol, n-butanol, isobutanol, n-pentanol, n-hexanol, n-heptanol, n-octanol, nonanol, dodecanol, tetrahydrofurfurol, ethoxyethanol, propoxyethanol, methoxyethanol, methoxybutanol, trialkylsilylethanol, trialkylsilylpropanol, trialkylsilylbutanol, benzylalcohol, propargyl alcohol, phenyl-2-ethanol, cyclohexenylethanol.

The compounds of formula (III) may be those wherein R = R' =H, or those with R = H and R' = alkyl such as for example methyl or ethyl.

The compound of formula (III) may have a biocarbon content equal to or higher than 0%, more preferably equal to or higher than 24%, and even more preferably equal to or higher than 49%.

In a preferred embodiment, the compound of formula (III) does not derive from aspartic acid derivatives.

*Process P-B*

The process P-B of preparation of a compound of formula (II) may comprise:

- contacting a compound of formula (V) with a compound of formula (IV) as defined above under conditions sufficient to yield a compound of formula (II):

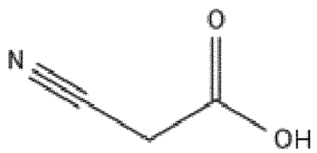 (V) 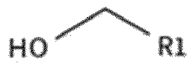 (IV)

- optionally separating therefrom the compound of formula (II).

The reaction is well known in this field and may be carried out as disclosed in Chem Sci., 2021, 12, 10259-10265 (Wang et al.). It corresponds typically to an esterification of cyanoacetic acid with an alcohol.

The molar ratio of compound of formula (IV) to compound of formula (V) may vary from 1.0 to 20, preferably from 5.0 to 10.

Preferably, the reaction is carried out in presence of an acid. Esterification acid catalysts are well known to the man skilled in the art. They can be either Lewis or Bronsted acids. The acid may be selected from the group consisting of Sulfuric Acid, p-toluene Sulfonic Acid, Scandium Triflate, Methanesulfonic Acid, Dodecylbenzene Sulfonic Acid, Boron Trifluoride, Phosphoric Acid, Perchloric Acid, Hydrochloric Acid. Also heterogeneous catalysts may be used, for example: Zirconium Tetrasulfate, Amberlyst 46, Acid-modified Montmorillonite (K10) clay, and Tungstated zirconia.

The reaction may be carried out at a temperature ranging from 25°C to 120°C, preferably from 50°C to 100°C.

Preferably, the process P-B of preparation of a compound of formula (II) comprises a step of optionally separating the compound of formula (II) from the reaction medium obtained in the first step.

The process P-B preferably comprises:

- a step of concentration of the reaction medium under reduced pressure;
- a step of adding an organic solvent and an aqueous solution to the residue obtained, then ;
- a step of decantation, recovery of the organic phase, and evaporation of the organic solvent to obtain a compound of formula (II).

The compound of formula (V) may be petroleum-based, or at least partially biobased or fully biobased (biocarbon content of 100%).

All the description, embodiments and preferred embodiments disclosed above for compound of formula (IV) (for example in paragraph Process P-A) applies herein for process P-B, and are not reiterated.

The compound of formula (V) may be commercially available for example by Sigma-Aldrich.

The compound of formula (V) may also be obtained from a process comprising contacting a compound of formula (VI) with potassium cyanide (KCN) or sodium cyanide (NaCN) (preferably potassium cyanide) under conditions sufficient to yield a compound of formula (V):

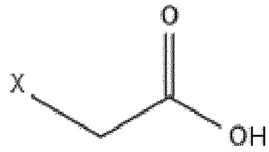
(VI)

wherein X represents a halogen atom, preferably Br or Cl, more preferably Br.

The potassium cyanide (KCN) or sodium cyanide (NaCN) may optionally be partially biobased, or even fully biobased. Biobased potassium cyanide can be obtained in two steps. The first step is the andrussow oxidation of biomethane (Engie) to obtain hydrogen cyanide HCN. (Andrussow L (1935). "The catalytic oxydation of ammonia-methane-mixtures to hydrogen cyanide". Angewandte Chemie. 48 (37): 593-595) Then biobased HCN is reacted with potassium hydroxide (KOH) to form biobased KCN. Pradyot Patnaik. Handbook of Inorganic Chemicals. McGraw-Hill, 2002, ISBN 0-07-049439-8.

The P-B reaction may be carried out typically as described in Chemistry, a European journal, vol 24, issue 71, p. 18903-18906; or CN2015-10810110 or CN2016-10662675.

The reaction may be carried out in basic medium, with a pH higher than 6, preferably higher than 7.

The reaction may be carried out in an aqueous basic solution, for example aqueous solution of $Na_2CO_3$ or $NaHCO_3$.

The reaction may last from 3 minutes to 24h.

The reaction may be carried out at an overpressure (vs ambient atmosphere) ranging from 0.01 to 1 MPa, preferably at 0.2 MPa.

The reaction may be carried out at a temperature ranging from 23°C to 100°C.

The process of preparation of a compound of formula (V) preferably comprises an isolation of the compound of formula (V), preferably comprising:

- an acidification step with an acid, for example HCl;
- an optional step of evaporation of water;
- an optional step of extraction with an organic solvent, preferably ether;
- an optional filtration step to recover the compound of formula (V).

The compound of formula (VI) may be petroleum-based, or partially biobased or even fully biobased (biocarbon content of 100%).

The compound of formula (VI) may be commercially available from Sigma-Aldrich such as for example Bromoacetic acid.

The compound of formula (VI) may also be obtained from a process comprising contacting a compound of formula (VII) with a halogenating agent H under conditions sufficient to yield a compound of formula (VI):

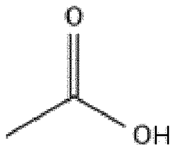
(VII)

The reaction may be carried out typically as described in Asian Journal of Chemistry, 30(10), 2310-2316 (2018).

The halogenating agent H may be selected from $PBr_3$, $Br_2$, $Cl_2$ and $PCl_5$, and mixtures thereof.

The compound of formula (VII) may be one commercialized by Sekab (acetic acid, CAS = 64-19-7) which is fully biobased (biocarbon content = 100%).

The compound of formula (VII) may be petroleum-based or at least partially or fully biobased (biocarbon content of 100%).

*Process P-C*

The process P-C of preparation of a compound of formula (II) may comprise:

- contacting a compound of formula (II') with a compound of formula (IV) as defined above, under conditions sufficient to yield a compound of formula (II):

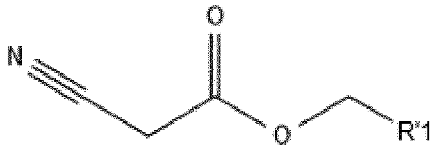
(II')

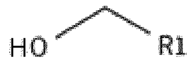
(IV)

wherein R'1 is H or an organic moiety different from R1, and R1 is as defined above;

- optionally separating therefrom the compound of formula (II).

This reaction is typically a transesterification reaction well known in the art. It can for example be carried out as described in Zhanjie Zazhishe (2014), 35 (2), 67-68, 79.

The reaction may be carried out at a temperature ranging from 80 °C to 120 °C.

The reaction may be carried out in presence of a catalyst, for example selected from the group consisting of Titanium Isopropoxide, Aluminum Isopropoxide, Tributyltin Alkoxides, Dibutyltin Oxide, Tin Acetylacetonate.

The process of preparation P-C preferably comprise a step of separating the compound of formula (II). It is preferably carried out by distillation, in particular under reduced pressure.

All the description, embodiments and preferred embodiments disclosed above for compound of formula (IV) (for example in paragraph Process P-A and P-B) applies herein for process P-C, and are not reiterated.

The compound of formula (II') may be petroleum-based, or partially biobased, or fully biobased.

The compound of formula (II')

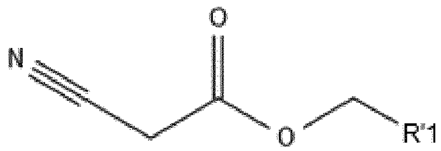
(II')

wherein R1' is as defined above, may have a biocarbon content equal to or higher than 20%, preferably equal to or higher than 25%, more preferably equal to or higher than 41%, even more preferably equal to or higher than 50%, and even more advantageously equal to or higher than 55%.

Preferably, R'1 is selected from the group consisting of: H, alkyl, halogenated alkyl, alkyl silane, acetoxy silane, alkenyl, alkynyl, aryl, heteroaryl, alkaryl, aralkyl, cycloalkyl, cycloalkenyl, heterocycloalkyl, alkoxyalkyl, acrylic ester moiety, oxetane moiety, epoxy moiety, carboxylic ester moiety.

The alkyl, alkenyl, aryl, alkynyl, heteroaryl, alkaryl, aralkyl, cycloalkyl, cycloalkenyl, heterocycloalkyl, alkoxyalkyl may be substituted, for example by at least one substituent selected from the group consisting of: alkyl, aryl, alkenyl, OH, amine, amide, halogen atom, - $CF_3$, and mixtures thereof.

The compound of formula (II') is preferably selected from methylcyanoacetate, ethylcyanoacetate, n-propylcyanoacetate, n-butylcyanoacetate, isobutylcyanoacetate, n-heptylcyanoacetate, n-octylcyanoacetate, n-hexylcyanoacetate, n-pentylcyanoacetate, nonylcyanoacetate, dodecyanoacetate, tetrahydrofurfuryl cyanoacetate, 2-ethoxyethylcyanoacetate, 2-methoxyethylcyanoacetate, 2-methoxybutylcyanoacetate, 2-propoxyethylcyanoacetate, propargylcyanoacetate, 2-phenylethylcyanoacetate, cyclohexenylcyanoacetate, trialkylsilylethylcyanoacetate, trialkylsilylpropylcyanoacetate, trialkylsilylbutylcyanoacetate, benzylcyanoacetate.

More preferably, the compound of formula (II') is methylcyanoacetate.

*Process P-D*

The process P-D of preparation of a compound of formula (II) may comprise:

- contacting a compound of formula (VIII) with a compound of formula (IV) under conditions sufficient to yield a compound of formula (II):

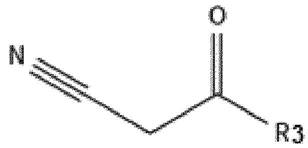 (VIII) 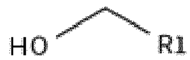 (IV)

wherein R1 is as defined above for formula (I), namely H or an organic moiety,
wherein R3 is Br, Cl or I;

- optionally separating therefrom the compound of formula (II).

Preferably, the reaction is carried out in presence of an acid. Esterification acid catalysts are well known to the man skilled in the art. They can be either Lewis or Bronsted acids. The acid may be selected from the group consisting of Sulfuric Acid, p-toluene Sulfonic Acid, Scandium Triflate, Methanesulfonic Acid, Dodecylbenzene Sulfonic Acid, Boron Trifluoride, Phosphoric Acid, Perchloric Acid, Hydrochloric Acid. Also heterogeneous catalysts may be used, for example: Zirconium Tetrasulfate, Amberlyst 46, Acid-modified Montmorillonite (K10) clay, and Tungstated zirconia.
The compound of formula (VIII) may be used in excess to either or both the compound of formula (IV) and the acid.
The reaction may be carried out at a temperature ranging from 25°C to 120°C, preferably from 50°C to 100°C.
Preferably, the process P-D of preparation of a compound of formula (II) comprises a step of optionally separating the compound of formula (II) from the reaction medium obtained in the first step.
The process P-D preferably comprises:

- a step of concentration of the reaction medium under reduced pressure;
- a step of adding an organic solvent and an aqueous solution to the residue obtained, then ;
- a step of decantation, recovery of the organic phase, and evaporation of the organic solvent to obtain a compound of formula (II).

The compound of formula (VIII) may be petroleum-based, or partially biobased or fully biobased (biocarbon content of 100%).
All the description, embodiments and preferred embodiments disclosed above for compound of formula (IV) (for example in paragraph Process P-A) applies herein for process P-D, and are not reiterated.

*Process P-E*

The process P-E of preparation of a compound of formula (II) may comprise:

- contacting a compound of formula (IX) with a compound of formula (IV) under conditions sufficient to yield a compound of formula (II):

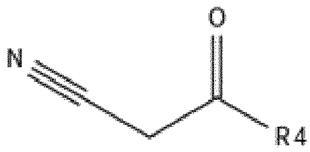 (IX) 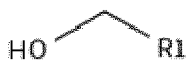 (IV)

wherein R1 is as defined above for formula (I), namely H or an organic moiety,
wherein R4 is -O-C(=O)-R4, with R4 being selected from alkyl or aryl;

- optionally separating therefrom the compound of formula (II).

Preferably, the reaction is carried out in presence of an acid. Esterification acid catalysts are well known to the man skilled in the art. They can be either Lewis or Bronsted acids. The acid may be selected from the group consisting of Sulfuric Acid, p-toluene Sulfonic Acid, Scandium Triflate, Methanesulfonic Acid, Dodecylbenzene Sulfonic Acid, Boron Trifluoride, Phosphoric Acid, Perchloric Acid, Hydrochloric Acid. Also heterogeneous catalysts may be used, for example: Zirconium Tetrasulfate, Amberlyst 46, Acid-modified Montmorillonite (K10) clay, and Tungstated zirconia.
The compound of formula (IX) should be used in excess to either or both the compound of formula (IV) and the acid.
The reaction may be carried out at a temperature ranging from 25°C to 120°C, preferably from 50°C to 100°C.

Preferably, the process P-E of preparation of a compound of formula (II) comprises a step of optionally separating the compound of formula (II) from the reaction medium obtained in the first step.
The process P-E preferably comprises:

- a step of concentration of the reaction medium under reduced pressure;
- a step of adding an organic solvent and an aqueous solution to the residue obtained, then ;
- a step of decantation, recovery of the organic phase, and evaporation of the organic solvent to obtain a compound of formula (II).

The compound of formula (VIII) may be petroleum-based, or partially biobased or fully biobased (biocarbon content of 100%).
All the description, embodiments and preferred embodiments disclosed above for compound of formula (IV) (for example in paragraph Process P-A) applies herein for process P-E, and are not reiterated.
Preferably, the compound of formula (II) does not derive from aspartic acid derivatives.
In a preferred embodiment, the compound of formula (II) as defined above is obtained from process P-B or P-C.
In one preferred embodiment, the compound of formula (II) wherein R1 = methyl is obtained by a process P-B comprising:

- contacting a compound of formula (V) with a compound of formula (IV) wherein R1 = methyl above under conditions sufficient to yield a compound of formula (II):

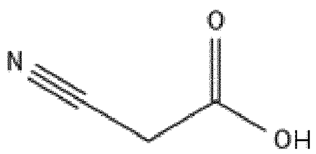 (V) 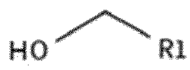 (IV)

- optionally separating therefrom the compound of formula (II),

wherein :

- the compound of formula (IV) has a biocarbon content equal to or higher than 20%, more preferably equal to or higher than 50%, even more preferably equal to or higher than 80%, and more advantageously equal to or higher than 99%;
- the compound of formula (V) is petroleum-based, or partially biobased or fully biobased.

In another preferred embodiment, the compound of formula (II) wherein R1 = hexyl or tetrahydrofuryl is obtained by a process P-C comprising:

- contacting a compound of formula (II') with a compound of formula (IV) wherein R1 = hexyl or tetrahydrofuryl, under conditions sufficient to yield a compound of formula (II):

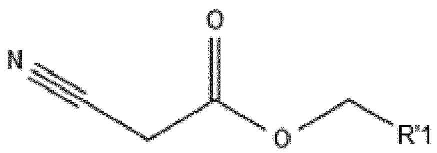 (II') 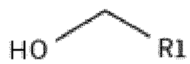 (IV)

  wherein R'1 is H or an organic moiety different from R1,
- optionally separating therefrom the compound of formula (II),

wherein :

- the compound of formula (IV) has a biocarbon content equal to or higher than 20%, more preferably equal to or higher than 50%, even more preferably equal to or higher than 80%, and more advantageously equal to or higher than 99%;

- the compound of formula (II) is petroleum-based, or partially biobased or fully biobased.

## USES

The present invention concerns the use of a compound of formula (I) as described above, for preparing a composition, preferably an adhesive composition.

The present invention also concerns a composition, more preferably an adhesive composition comprising the compound of formula (I) as described above.

All the description, embodiments, and preferred embodiments disclosed above for the compound of formula (I) (for example in paragraph COMPOUNDS) apply herein for the uses and compositions, and are not reiterated.

The composition may be 1K or 2K composition.

Preferably, the adhesive composition comprising the compound of formula (I) as disclosed above, has a biocarbon content equal to or higher than 20%, even more preferably higher to or equal to 50%. The biocarbon content is measured as defined above.

In a preferred embodiment, the composition comprise more than 80% of compound(s) of formula (I) as defined above, even more preferably equal to or more than 85%, more preferably equal to or more than 90% by weight based on the total weight of the composition.

This type of composition, comprising cyanoacrylate, is well known and can comprise any ingredients and/or additives well known in this field.

The composition may comprise an acidic inhibitor.

The acid inhibitors are inhibitors of the anionic polymerization. They may be selected from the group consisting of Bronsted acids, Lewis acids, acids provided from an acidic ion exchange material or photoacids obtained from photoacid generators (also known as PAGs), and mixtures thereof; preferably from Bronsted acids, Lewis acids, and mixtures thereof. The acid inhibitors are preferably selected from the group consisting of methanesulfonic acid, p-toluenesulfonic acid, p-toluenesulfonic acid anhydride, hydrofluoric acid, boron trifluoride, boron trifluoride etherate complex, boron trifluoride dihydrate, sulfur dioxide, sulfur trioxide, tin (IV) chloride, iron (III) chloride, citric acid, trimethylsilyl triflate, and mixtures thereof; more preferably from methanesulfonic acid, hydrofluoric acid, boron trifluoride, boron trifluoride etherate complex, sulfur dioxide, and mixtures thereof.

The composition may comprise from 0% to 0.1 %, preferably from 0.0001% to 0.05 %, more preferably from 0.0005 to 0.01 % by weight, of acidic inhibitor(s), based on the total weight of said composition.

The expressions "radical stabilizer" and "radical stabilizing agent" are presently used interchangeably.

The composition may comprise a radical stabilizer.

The radical stabilizer is a free radical polymerization inhibitor. It is preferably selected from the group consisting of hindered phenolic or polyphenolic compounds; preferably from hydroquinone, hydroquinone monomethyl ether, mono-tertiary-butyl hydroquinone, 2,5-ditertiary-butyl-hydroquinone, p-methoxyphenol, hydroxyanisole, butylated hydroxyanisole, hydroxyanisol butyl ether, 2,6-di-tert-butyl-p-cresol, 2,2'-methylene-bis-(6-tert-butyl-4-methylphenol), p-tert-butyl catechol, 1,3,5-trimethyl-2,4,6-tris(3,5-di-tert-butyl-4-hydroxybenzyl) benzene, hydroxytoluene butyl ether, and mixtures thereof; more preferably from hydroquinone monomethyl ether, 2,2'-methylene-bis-(6-tert-butyl-4-methylphenol), and mixtures thereof.

The composition may comprise from 0.01 to 0.7 % by weight, preferably from 0.01 to 0.5% by weight, more preferably from 0.01 to 0.4 % by weight, of a radical stabilizer, based on the total weight of said composition.

The expressions "thickeners" or "thickening agents" are presently used interchangeably.

The composition may comprise a thickener.

Thickeners are particularly suitable for controlling and/or increasing the viscosity of the cyanoacrylate component, considering that cyanoacrylate-based monomers usually have a water-like viscosity at ambient temperature. A suitable thickener or thickening agent for the first part of the composition can be selected from those that are compatible with the host monomers. Examples of such thickeners include poly(meth)acrylates, acylated cellulose polymers (for example, cellulose acetate), polyvinyl acetates, partially hydrolysed polyvinyl acetates, polyvinylpyrrolidones, polyoxylates, polycaprolactones, polycyanoacrylates, vinyl acetate copolymers (for example, with vinyl chloride), copolymers of (meth)acrylates with butadiene and styrene, copolymers of vinyl chloride and acrylonitrile, copolymers of ethylene and vinyl acetate, poly[butyleneterephthalate-co-polyethyleneglycolterephthalate, copolymers of lactic acid and caprolactone, and mixtures thereof.

These thickeners are well known to the skilled in the art and have been described in the prior art. Preferably, the thickener is selected from the group consisting of poly(meth)acrylates, polymethyl(meth)acrylate, polyvinylpyrrolidones, polyvinyl acetates, partially hydrolysed polyvinyl acetates, vinyl acetate copolymers, acrylated cellulose polymers, and mixtures thereof. A suitable thickener for the cyanoacrylate component may be, for example, polymethylmethacrylate (for example Degacryl® M 449, Evonik), copolymers of vinyl acetate and vinyl alcohol (for example Levamelt® 900, Lanxess), copolymers of vinyl chloride and vinyl acetate (for example, Vinnol® H 40-60, Wacker), copolymers

of ethylene, vinyl acetate, and esters or partial esters of maleic acid (for example, Vamac® G, DuPont), and mixtures thereof.

The composition may comprise from 0% to 10 % by weight, preferably from 3 to 8 % by weight, more preferably from 4 to 7 % by weight, of a thickener, by total weight of said composition.

The expressions "tougheners" or "toughening agents" are presently used interchangeably.

The composition may comprise a toughener.

The toughener may be selected from the group consisting of block copolymers (for example,polymethylmethacrylate-co-polybutylacrylate-co-polymethylmethacrylate), elastomeric rubbers, elastomeric polymers, liquid elastomers, polyesters, acrylic rubbers, butadiene/acrylonitrile rubber, Buna rubber, polyisobutylene, polyisoprene, natural rubber, synthetic rubber (for example, styrene/butadiene rubber (SBR)), polyurethane polymers, ethylene-vinyl acetate polymers(LEVAMELT 700 and 900), fluorinated rubbers, isopreneacrylonitrile polymers, chlorosulfonated polyethylenes, homopolymers of polyvinyl acetate, block copolymers, core-shell rubber particles (for example, commercially available from Arkema under the trade name Clearstrength® XT100 and XT151), and mixtures thereof.

The composition may comprise from 1 to 20 % by weight, preferably from 5 to 12 % by weight, of a toughener, based on the total weight of said composition.

The composition may comprise an accelerating agent.

The expressions "accelerator" or "accelerating agent" are presently used interchangeably.

The accelerator may be selected from the group consisting of crown ethers (for example, 15-crown-6 ether, 15-crown-5 ether, 18-crown-6 ether, dibenzo-18-crown-6 ether, and mixtures thereof), cyclodextrins, silylated crown ethers, calixarenes tetra-t-butyl esters, dimethyl ethers of PEG 400, PEG 500, PEG 600 and PEG 1000, and mixtures thereof; preferably the accelerator is a crown ether; more preferably the accelerator is selected from the group consisting of 18-crown-6 ether, dibenzo-18-crown-6 ether and mixtures ; still more preferably the accelerator is dibenzo-18-crown-6.

The composition may comprise from 0% to 0.8 % by weight, preferably from 0.05 to 0.5 % by weight, of an accelerator, based on the total weight of said composition.

The composition may comprise an adhesion promoter.

The expression "adhesion promoter" and "adhesion promoting agent" are presently used interchangeably.

The composition may comprise an adhesion promoter for glass, ceramics, porcelain, plastics and/or metal, for example, alkoxysilane compounds. Analogously to the cyanoacrylate component, the additional component may comprise an adhesion promoter for glass, ceramics, porcelain, plastics and/or metal such as, for example, alkoxysilane compounds, citric acid, lithium tetrafluoroborate, lithium hexafluorophosphate, an aromatic carboxylic acid or anhydride or an $\alpha$-substituted acrylic acid, optionally in form of anhydride. Among the acids and anhydrides can be mentioned trimellitic acid, trimellitic anhydride, pyromellitic acid, pyromellitic anhydride, 3,3',4,4'-benzophenonetetracarboxylic acid dianhydride, itaconic acid, itaconic anhydride, and 3-buten-1,2,3-tricarboxylic acid.

The composition may comprise from 0% to 0.1 % by weight, preferably from 0.06 to 0.08 % by weight, of an adhesion promoter, based on the total weight of said composition.

The composition may comprise a thixotropic agent.

The thixotropic agent may be an organic thixotropic agent, an inorganic thixotropic agent; preferably a thixotropic agent selected from the group consisting of hydrogenated castor oil (EFKA RM 1900), hydrogenated castor oil modified by reaction with an amine, polyamides, silica, and mixtures thereof.

The silica may be fumed silica; preferably silica may be selected from the group consisting of fumed silica, hydrophobic fumed silica, hydrophilic fumed silica and precipitated silica, and mixtures thereof; more preferably from hydrophobic fumed silica. Hydrophobic fumed silica may also act as a filler. A hydrophobic fumed silica is commercially available under the commercial denomination Aerosil® R202 from Evonik.

The composition may comprise from 0% to 10 % by weight, preferably from 2.5 to 8 % by weight, more preferably from 3 to 6 % by weight, of a thixotropic agent, based on the total weight of said composition.

The composition may comprise a photoinitiating system *i.e.,* a source of photoinitiators. Photoinitiators promote photopolymerization. The terms "photoiniator", "photoinitiator system", "photoinitiating system" and "source of photoinitiators" are used interchangeably presently. A suitable photoinitiating system, part of which comprises radical photoinitiators, is disclosed in the PCT application WO 2017/021785 A1. The photoinitiating system is particularly an anionic or zwitterionic photoinitiating system i.e., a photoinitiating system, which is suitable for promoting an anionic or zwitterionic polymerization. The expressions "an anionic or zwitterionic photoinitiating system" and "a photoinitiating system suitable for promoting an anionic or zwitterionic polymerization" are used interchangeably.

A suitable photoinitiating system (source of photoinitiators) comprises a metallocene component (also called "synergist"); preferably a metallocene compound selected from the group consisting of 'sandwich compounds' such as ferrocene compounds, ruthenocene compounds, bis(cyclopentadieneyl) osmium compounds their derivatives thereof, and mixtures thereof; preferably the metallocene compound is a ferrocene compound, its derivatives thereof, and mixtures thereof. The ferrocene compound may be:

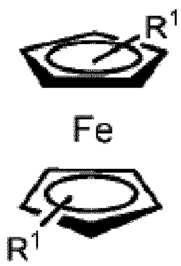

wherein $R^1$ is a hydrogen or a $C_{1-4}$ alkyl group, and wherein one or more $R^1$ are present in one or both rings. Suitable ferrocene compounds are disclosed in US patents 5,824,180 and 6,503,959.

This suitable photoinitiating system (source of photoinitiators) also comprises an additional photoinitiator.

The additional photoinitiator may be selected from the group consisting of phenyl-substituted acyl phosphines, alpha diketones, thioxanthones, alpha-hydroxy ketones, benzyldimethylketals, phenylglyoxylates, camphorquinone, acylgermane compounds, dialkylsilylglyoxylates, and mixtures thereof. The additional photoinitiators are particularly useful for increasing the speed of the polymerization of the cyanoacrylate-based monomers.

The acylgermane compound may be one of the following:

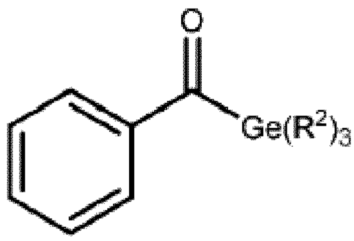

wherein $R^2$ is a methyl or a phenyl group;

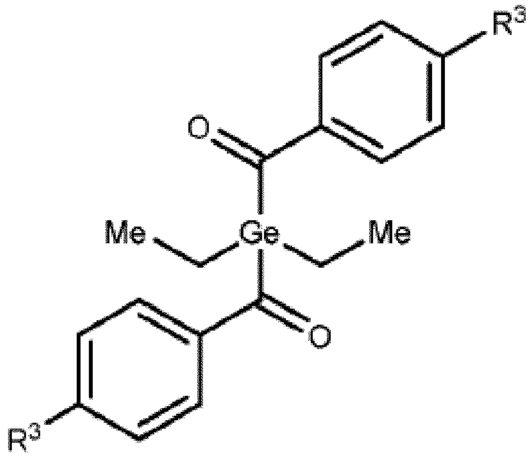

wherein $R^3$ is hydrogen or a methoxy group; and,
mixtures thereof.

Suitable additional photoinitiators are commercially available under the denominations Irgacure® 819 (BASF) or Darocur® TPO (BASF) for phenyl-substituted acyl phosphines; Irgacure® 184/500/2959 (BASF) or Darocur® 1173 (BASF) for alpha-hydroxy ketones; Irgacure® 651 (BASF) for benzyldimethylketals, Irgacure® 754 (BASF) or Darocur® MBF (BASF) for phenylglyoxylates; from Sigma-Aldrich Merck for camphorquinone; under the denomination Ivocerin™ from Ivoclar KGaA& AC Co. for acyl germane compounds; and from Sigma-Aldrich Merck for dialkylsilylglyoxylates.

## OTHERS

The present invention also concerns the use of at least one raw material R having a biocarbon content higher than 0%, for the preparation of a compound of formula (I):

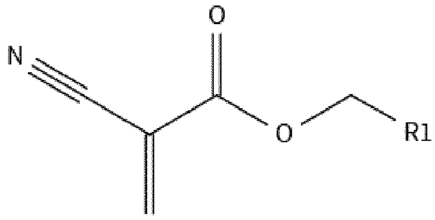

(I)

wherein R1 is H or an organic moiety, said compound of formula (I) having a biocarbon content equal to or higher than 20%.

All the description, embodiments and preferred features for compound of formula (I) described above (in paragraph "COMPOUNDS") apply to the use herein, and is not reiterated.

The raw material R preferably has a biocarbon content equal to or higher than 41%, more preferably equal to or higher than 50%. Even more preferably, the raw material R has a biocarbon content equal to or higher than 65%, and more preferably equal to 100%.

The raw material R may be selected from the group consisting of: acetic acid, ethanol, n-heptanol, isobutanol, n butanol, 1,10 decanediol, tetrahydrofurfuryl ethanol, preferably it is ethanol and n-heptanol, even more preferably n-heptanol.

The present invention relates to a compound of formula (I) as defined herein, said compound of formula (I) deriving from an alcohol compound having a biocarbon content equal to 100%, said alcohol being preferably ethanol or heptanol.

All the description, embodiments and preferred features for compound of formula (I) described above (in paragraph "COMPOUNDS") apply herein, and is not reiterated.

The compound of formula (I) advantageously derived at least in part from raw materials of renewable origin.

The compound of formula (I) advantageously exhibits good adhesive properties, good fixturing times.

The compound of formula (I) can be advantageously produced industrially with good yields.

The compound of formula (I) wherein R1 is C6 alkyl group, (and/or the composition comprising it) advantageously exhibits in addition high resistance to hot water (for example at 60°C).

The compound of formula (I) wherein R1 is C6 alkyl group (and/or the composition comprising it) advantageously exhibits a strength retention of more than 80% after full submersion in hot water (at 60°C) for 3 to 10 days, according to the test method disclosed hereafter in the experimental part.

The compound of formula (I) wherein R1 is C6 alkyl group (and/or the composition comprising it) advantageously exhibits a very low blooming, according to the test method disclosed hereafter in the experimental part.

The ranges disclosed in this description include both the lower and the upper limit thereof. For example, the expressions "ranging from x to y" or "between x and y" comprises the limits x and y.

## Examples

The following ingredients were used in the examples:

- acetic acid commercialized by Sekab, biobased acetic acid
- cyanoacetic acid, supplied by Sigma-Aldrich
- bromoacetic acid, supplied by Sigma-Aldrich
- Sulfuric Acid, Hydrochloric Acid, supplied by Scharlab
- Phosphorous Tribromide supplied by Sigma-Aldrich
- Bromine supplied by Sigma-Aldrich
- Sodium Carbonate supplied by Sigma-Aldrich
- Sodium Thiosulfate, supplied by Sigma-Aldrich
- Diethyl Ether, Toluene, Ethyl Acetate, supplied by Scharlab
- Potassium Cyanide supplied by Sigma-Aldrich
- biobased Ethanol supplied by Myhome aromas at a purity of 99,5 %
- petroleum-based Ethanol, supplied by Ineos
- Methylene Diacetate, supplied by CMC
- Linear dodecylbenzene Sulfonic Acid, LABSA (CAS 27176-87-0) supplied by Henkel
- 2-Methylpiperazine supplied by Sigma-Aldrich
- Piperidine, supplied by Sigma-Aldrich
- 2,2'-Methylenebis(4-methyl-6-tert-butylphenol) supplied by TCI

- Paraformaldehyde, supplied by Sigma-Aldrich
- Piperidine supplied by Sigma-Aldrich
- biobased heptanol supplied by Arkema Oleris at a purity of >99%
- Methyl Cyanoacetate, supplied by Hebei
- Titanium Isopropoxide supplied by Sigma-Aldrich
- p-Toluene Sulfonic Acid, supplied by Sigma-Aldrich
- Phosphorous Pentoxide, supplied by Sigma-Addrich
- Hydroquinone, supplied by TCI
- biobased tetrahydrofurfuryl alcohol supplied by Pennakem
- Dibenzo-18-Crown-6 DBC (18-c-6) from Ferak Berlin GmbH
- EFKA RM 1900 from BASF (100% biobased as analysed by ASTM D6866)
- Butylhydroxyanisole (BHA) from TCI
- Vinnol H40/60 from Wacker
- Tetrahydrofurfuryl alcohol (Viridisol® T, Pennakem),
- MSA from sigma Aldrich
- $SO_2$ from carburos metallicos

Among those ingredients, n-heptanol (Arkema Oleris), Ethanol (Myhome aromas), acetic acid (Sekab), Tetrahydrofurfuryl alcohol (Viridisol® T, Pennakem), EFKA RM 1900 are biomaterial (biocarbon content of 100%).

The biomaterial content or biocarbon content is determined by using the standards ASTM D 6866 (ASTM D 6866-21, method B) and ASTM D 7026 (ASTM D 7026-04). The ASTM D 6866 standard is "Determining the Biobased Content of Natural Range Materials Using Radiocarbon and Isotope Ratio Mass Spectrometry Analysis", while the ASTM D 7026 standard is "Sampling and Reporting of Results for Determination of Biobased Content of Materials via Carbon Isotope Analysis". The second standard makes reference in its first paragraph to the first standard.

The first standard describes a test for measuring the $^{14}C/^{12}C$ ratio of a sample and compares it with the $^{14}C/^{12}C$ ratio of a reference sample of 100 % renewable origin, to give a relative percentage of C of renewable origin in sample. The standard is based on the same concepts as $^{14}C$ dating, but without applying the dating equations.

The ratio thus calculated is referred to as the "pMC" (percent Modern Carbon). If the material to be analyzed is a mixture of biomaterial and fossil material (without radioactive isotope), then the pMC value obtained is directly correlated with the quantity of biomaterial present in the sample.

A C-14 modern standard (Oxalic Acid II) represents what modern (living plant) C-14 activity was in 1950 which has been set as the standard for 100% biobased activity. A background level is also established which is subtracted from the activity measured. This activity of the sample is compared to the activity of the Oxalic Acid II. This will give the pMC or percent of modern carbon. If there is a mixture of modern carbon and fossil carbon in the sample, then the resulting pMC (example 85 pMC) is indicating that we measured 85 percent of the activity of the modern standard. The sample would be reported as 85% biobased carbon. Due to nuclear tests in the atmosphere in the 1950's and 1960's C-14 was artificially created in the atmosphere. At one point it was nearly 200% of natural activity. Over time, with the dilution of fossil fuel $CO_2$ being put into the atmosphere this activity has been diluted such that today the C-14 activity in the atmosphere is back to the same level as the modern standard. There is an atmospheric correction factor that is used to account for these changes in the atmospheric C-14 activity. This atmospheric correction factor (REF) was used as following:

$$\text{Biocarbon content} = \text{pMC} / (\text{REF}),$$

with REF = 1.000

The precision on the biocarbon content % is of +/- 3% (absolute).

**Example 1 : preparation of ethylcyanoacrylate (process P1)**

Example 1a : process of preparation of bromoacetic acid (corresponding to a compound of formula (VI))

This step was adapted from a previously reported procedure (T.M. Werkhoven et al., Eur. J. Org. Chem. 1999, 2909-2914). A mixture of biosourced acetic acid (4.0 g, Sekab) and $PBr_3$ (18 g) were mixed at 0°C; then bromine (24.4 g) was added. After the HBr evolution had ceased, the reaction mixture was heated at 75 °C for 2.5 h. Reaction was quenched with water. Diethyl ether and $Na_2S_2O_3$ solution were added. The water layer was extracted with diethyl ether. Evaporation of the solvent yielded 9.11 g (98% yield) of biobased bromoacetic acid.

Example 1b : process of preparation of cyanoacetic acid (corresponding to a compound of formula (V))

This step was adapted from a previously reported procedure (J. Kremser et al., Chem. Comm. 2017, 53(96) 12938-12941). Bromoacetic acid obtained in example 1a (6.99 g) was dissolved in a solution of $Na_2CO_3$ (pH 11). An aqueous solution of potassium cyanide (3.27 g) was poured into the reaction mixture. The mixture was heated to 80°C for 2 hours and then stirred for 15 more hours at room temperature (23°C). Concentrated HCl (10 mL) was added until pH 1 was reached; the solvent was removed and solid residue was extracted with diethyl ether. The remaining salt was filtered and washed with additional diethyl ether. The filtrate was evaporated and biobased cyanoacetic acid was obtained (4.28 g; 100% yield). This step was repeated several times to obtain a higher quantity of biobased cyanoacetic acid.

Example 1c : process of preparation of cyanoacetate (corresponding to a compound of formula (II))

A mixture of Cyanoacetic Acid obtained in example 1b (50.0 g), biosourced EtOH (200 ml, Myhome aromas) and concentrated $H_2SO_4$ (3.2 ml) was refluxed for 8 hours. The mixture was concentrated under reduced pressure and EtOAc and water were added to the residue. The organic phase was separated, and the aqueous layer was extracted with EtOAc. The combined organic extracts were dried and concentrated under reduced pressure. Biobased Ethyl Cyanoacetate (61.4 g, 92%) was obtained pure enough for the next step.

Example 1d : process of preparation of cyanoacrylate (corresponding to a compound of formula (I))

A Into a four-necked flask provided with a stirrer, a thermometer, a water trap and a dropping funnel were charged 60 parts of paraformaldehyde, 200 parts of toluene and 0.2 part of piperidine. To the mixture was added dropwise at 80° to 90° C. with stirring 314 parts of biobased ethyl cyanoacetate obtained in example 1c. After the completion of the dropwise addition, the mixture was allowed to react under reflux, while removing the water formed by the reaction, until all of the theoretical amount of water had been distilled out. The reaction mixture was then cooled down to room temperature. To the resulting condensation mixture having a viscosity of 200 centipoises was added 300 parts of a 1% aqueous solution of p-toluenesulfonic acid. The mixture was shaken at 70° C, and then allowed to stand, upon which it separated into two layers. The oil layer thereof was taken out.

The oil layer was distilled under reduced pressure to remove the toluene. To the condensation product left in the pot were added 3 parts of each of phosphorus pentoxide and hydroquinone. The mixture was subjected to depolymerization by heating at 150° to 200° C. under a pressure of 8 to 20 mmHg to obtain 266 parts (83% yield) of a crude monomer. The crude monomer had a purity of 96.8%.

After the addition of 0.5% of phosphorus pentoxide and 0.5% of hydroquinone to the crude monomer, the resulting mixture was redistilled to obtain 220 parts of biobased ethyl cyanoacrylate.

**Example 2** : **preparation of ethylcyanoacrylate (process P2)**

Example 2a: process of preparation of cyanoacetate (corresponding to a compound of formula (II))

A mixture of Cyanoacetic Acid (50.0 g, Sigma Aldrich), biobased EtOH (200 ml, Myhome aromas) and concentrated $H_2SO_4$ (3.2 ml) was refluxed for 8 hours. The mixture was concentrated under reduced pressure and EtOAc and water were added to the residue. The organic phase was separated, and the aqueous layer was extracted with EtOAc. The combined organic extracts were dried and concentrated under reduced pressure. Biobased ethyl cyanoacetate (61.4 g, 92%) was obtained pure enough for the next step.

Example 2b : process of preparation of cyanoacrylate (corresponding to a compound of formula (I))

A mixture of biobased Ethyl Cyanoacetate obtained in example 2a (59.2 g), Methylene Diacetate (138.2 g), Linear Alkyl Benzene Sulfonic Acid (20.9 g; LABSA, dodecylbenzene sulfonic acid), 2-Methylpiperazine (1.31 g) and 2,2'-Methylenebis(4-methyl-6-tert-butylphenol) (0.30 g) was heated to 125 °C for 2 hours. Desired product biobased ethyl cyanoacrylate was obtained by distillation at reduced pressure (52.3 g; 80% yield).

**Example 3** : **preparation of n-heptylcyanoacrylate**

Example 3a : preparation of n-heptyl cyanoacetate (corresponding to a compound of formula (II))

A mixture of biosourced n-heptanol (250 g, Arkema Oleris), Methyl Cyanoacetate (212 g, commercial from HEBEI) and Titanium Isopropoxide (0.61 g) was heated to 120 °C for 3 hours, under a residual pressure of 100 mbar.

When Methanol stripping was completed, reaction was stopped, and Biobased n-Heptyl Cyanoacetate was obtained by distillation at reduced pressure (360 g; 92% yield).

Example 3b : preparation of n-heptylcyanoacrylate (corresponding to a compound of formula (I))

A mixture of Biobased n-Heptyl cyanoacetate obtained in example 3a (300 g), Methylene Diacetate (432 g), Linear dodecyl Benzene Sulfonic Acid (65.5 g, LABSA), 2-Methylpiperazine (4.10 g) and 2,2'-Methylenebis(4-methyl-6-tert-butylphenol) (1.50 g) was heated to 125 °C for 2 hours. Desired product was obtained by distillation at reduced pressure (226 g; 76% yield). The resulting mixture was redistilled to obtain 180 g of biobased n-heptyl cyanoacrylate.

**Example 4 (comparative example) : preparation of ethylcyanoacrylate**

A mixture of Cyanoacetic Acid (50.0 g; supplied by Sigma-Aldrich, petroleum-based), petroleum-based EtOH (200 ml, Ineos) and concentrated $H_2SO_4$ (3.2 ml) was refluxed for 8 hours. The mixture was concentrated under reduced pressure and ethyl acetate and water were added to the residue. The organic phase was separated, and the aqueous layer was extracted with ethyl acetate. The combined organic extracts were dried and concentrated under reduced pressure. Ethyl Cyanoacetate (61.4 g, 92%) was obtained pure enough for the next step.

Into a four-necked flask provided with a stirrer, a thermometer, a water trap and a dropping funnel were charged 60 parts of paraformaldehyde, 200 parts of toluene and 0.2 part of piperidine. To the mixture was added dropwise at 80° to 90° C. with stirring 314 parts of ethyl cyanoacetate (obtained above in this example 4). After the completion of the dropwise addition, the mixture was allowed to react under reflux, while removing the water formed by the reaction, until all of the theoretical amount of water had been distilled out. The reaction mixture was then cooled down to room temperature. To the resulting condensation mixture having a viscosity of 200 centipoises was added 300 parts of a 1% aqueous solution of p-toluenesulfonic acid. The mixture was shaken at 70° C., and then allowed to stand, upon which it separated into two layers. The oil layer thereof was taken out.

The oil layer was distilled under reduced pressure to remove the toluene. To the condensation product left in the pot were added 3 parts of each of phosphorus pentoxide and hydroquinone. The mixture was subjected to depolymerization by heating at 150° to 200° C. under a pressure of 8 to 20 mmHg to obtain 266 parts (83% yield) of a crude monomer. The crude monomer had a purity of 96.8%.

After the addition of 0.5% of phosphorus pentoxide and 0.5% of hydroquinone to the crude monomer, the resulting mixture was redistilled to obtain 220 parts of ethyl cyanoacrylate.

**Example 5 : preparation of tetrahydrofurfuryl cyanoacrylate**

Example 5a: preparation of tetrahydrofurfuryl cyanoacetate (corresponding to a compound of formula (II))

A mixture of biosourced Tetrahydrofurfuryl alcohol (306 g, Viridisol® T, Pennakem), Methyl Cyanoacetate (297 g, commercial from Hebei) and Titanium Isopropoxide (0.853 g) was heated to 130 °C for 2 hours, under a residual pressure of 150 mbar. When Methanol stripping was completed, reaction was stopped, and biobased Tetrahydrofurfuryl Cyanoacetate was obtained by distillation at reduced pressure (383 g; 76% yield).

Example 5b: preparation of Tetrahydrofurfuryl cyanoacrylate (corresponding to a compound of formula (I))

A mixture of Biobased tetrahydrofurfuryl cyanoacetate obtained in example 5a (130 g), Methylene Diacetate (204 g), Linear Dodecyl Benzene Sulfonic Acid (30.9 g), 2-Methylpiperazine (1.93 g) and 2,2'-Methylenebis(4-methyl-6-tert-butylphenol) (0.65 g) was heated to 125 °C for 2 hours. Desired product was obtained by distillation at reduced pressure (64 g; 46% yield).

**Example 6: results**

The following Table highlights biocarbon content analysis and long term instant adhesive property of selected molecules, especially during shelf life (accelerated ageing of 3 days at 82ºC, simulating typical time before use of cyanoacrylate superglues).

Method for measuring the fixturing time FT :

Fixture time (FT) is the time at which an adhesive drop applied on a 250 mm2 surface is capable of supporting a 3 kg load for 10 seconds at room temperature (23 ± 2 ºC) under 50 % Relative Humidity (RH). FT was measured on

mild steel lapshear (MS). Those lapshears have a dimension of 100x25x1,5mm and were supplied by Rocholl.

The fixture time is measured after a 3 days 82ºC (3d82ºC) forced ageing in an aluminium uncoated tube. Indeed this accelerated ageing represents a good way to estimate the reactivity of the cyanoacrylate adhesive during its shelf life, when reaching final customers (3d82ºC corresponds to about 9-15 months depending on the formulation and packaging).

| Examples | Example/Supplier | Measured Biosourced Carbon Content (%)* | Theoretical Biosourced Carbon content (%)** | FT (MS) after 3d82ºC |
|---|---|---|---|---|
| Ethyl cyanoacrylate | Comparative example 4 | 0 | 0 | 10s |
| Biobased ethyl cyanoacrylate | Example 1 (invention) | 66 | 67 | 10s |
| Biobased ethyl cyanoacrylate | Example 2 (invention) | 34 | 33 | 15s |
| Biobased n-heptyl cyanoacrylate | Example 3 (invention) | 65 | 64 | 25 s |
| Biobased tetrahydrofurfuryl cyanoacrylate | Example 5 (invention) | 57 | 56 | 20 s |
| Biobased n-heptyl cyanoacetate | Intermediate compound prepared in example 3a | 70 | 70 | - |
| Biobased n-heptanol | Oleris-Arkema | 100 | 100 | - |
| Biobased ethanol | Myhome aromas | 100 | 100 | - |
| Ethanol (petroleum based) | Ineos | 0 | 0 | - |
| * Precision is +/- 3% (absolute)<br>** Ratio between Biobased carbons expected in a molecule and overall Carbons. | | | | |

Advantageously, each of the cyanoacrylate of examples 1, 2, 3 and 5 having a biocarbon content respectively of 66%, 34% 65% and 57%, exhibits good adhesion properties, even as good as petroleum-based corresponding cyanoacrylates : for example biobased cyanoacrylate of examples 1 and 2 exhibit a fast fixturing time (respectively 10s and 15s), whereas the same cyanoacrylate but petroleum-based (comparative example 4) provides quasi similar fixturing time 10s.

## Example 7 : preparation of adhesive compositions C1 and C2

The following C1 and C2 adhesive compositions were prepared as follows according to the nature and content of the ingredients disclosed in table 1: The monomers were directly mixed with the radical and acid stabilizing agents (except sulfur dioxide) in a high-density polyethylene container, the mixture was heated to approximately 60 °C, then the thickening agent was added under mechanical stirring until complete dissolution as was reported in WO2016038514. The mixture was left cool down to approximately 40 °C and the remaining components were added, including the sulfur dioxide if present.

| | Component | C1 (wt %) | C2 (wt%) |
|---|---|---|---|
| | N-heptylcyanoacrylate of example 3 (biocarbon content =65%) | Qsp 100% | Qsp 100% |
| | Butylhydroxyanisole (BHA) | 950 ppm | 950 ppm |
| | methane sulfonic acid | 1 ppm | 1 ppm |
| | Sulfur dioxide | 5 ppm | 5 ppm |
| | Vinnol H40/60 (thickener/toug hener) | 8 wt% | - |
| | EFKA RM 1900 (biobased thickener/toughener) | - | 5 wt% |
| | DBC (18-c-6) | 0.15 wt% | 0.15 wt% |
| | Biocarbon content of the compositions | 60% | 68% |

Tensile shear strength test data reflect bond strength measured in MPa after assembling overlapping lapshears (standard test pieces of grit blasted mild steel (GBMS)) with a contact area of 250 mm2, measured according to ASTM D1002. The bonded lapshears were first clamped and 'rested' for 24 h at room temperature (23°C ± 2°C) after assembly before testing.

The tests were carried out with an Instron tensile tester operating with a crosshead speed of 1.3 mm/min.

Testing for hot water resistance was conducted on assembled GBMS lapshear samples exposed to full submersion in hot water (at 60º C) for 3 days, and 10 days then rested for 24 hours at room temperature and destructively tested.

Blooming was qualitatively assessed by putting a drop of the adhesive composition on top of a black ABS in a 98%HR / 40ºC oven. The more the vapor generated polymerization around the drop the more the blooming.

| | blooming | 3d60ºC in water strength retention | 10d60ºC strength in water retention |
|---|---|---|---|
| C1 | Very low | 85% | 86% |

Composition C1 also advantageously exhibits extreme resistance to hot water (60°C) and very low blooming.

**Claims**

1. Compound of formula (I):

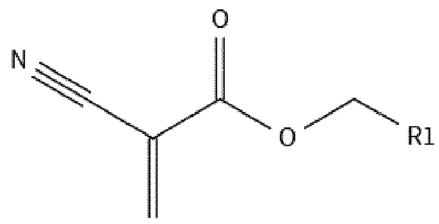

(I)

wherein R1 is H or an organic moiety, said compound of formula (I) having a biocarbon content equal to or higher than 20%.

2. Compound according to claim 1, having a biocarbon content equal to or higher than 25%.

3. Compound according to claim 1, having a biocarbon content equal to or higher than 41%, more preferably equal to or higher than 50%, and more preferably equal to or higher than 55%.

4. Compound according to any one of claims 1 to 3, wherein R1 is selected from the group consisting of: H, alkyl, halogenated alkyl, alkyl silane, acetoxy silane, alkenyl, alkynyl, aryl, heteroaryl, alkaryl, aralkyl, cycloalkyl, cycloalkenyl, heterocycloalkyl, alkoxyalkyl, acrylic ester moiety, oxetane moiety, epoxy moiety, carboxylic ester moiety.

5. Compound according to any one of claims 1 to 4, **characterized in that** it has one of the following formulae (I-A), (I-B), or (I-C)

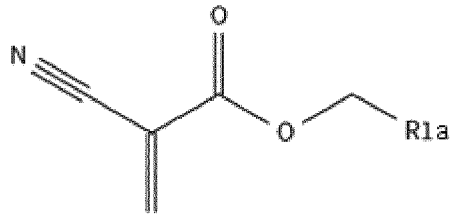

(I-A)

wherein R1a is alkyl,

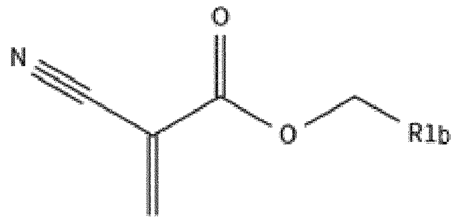

(I-B)

wherein R1b is selected from H, alkyl silane, acetoxy silane, alkenyl, alkynyl, aryl, heteroaryl, alkaryl, aralkyl, cycloalkyl, cycloalkenyl, heterocycloalkyl, alkoxyalkyl, oxetane moiety, epoxy moiety, preferably R1b being selected from cycloalkyl, aralkyl, heteroaryl, heterocycloalkyl, alkoxyalkyl;

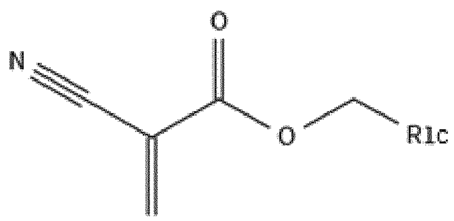

(I-C)

wherein R1c selected from acrylic ester moiety and carboxylic ester moiety;
the compound of formula (I) having preferably a formula (I-A) or (I-B).

6. Compound according to any one of claims 1 to 4, wherein R1 is selected from alkyl group and heterocycloalkyl.

7. Compound according to any one of claims 1 to 4 or 6, wherein R1 is a C1-C20 alkyl group, more preferably C1-C10 alkyl group, even more preferably C6 alkyl group.

8. Process of preparation of a compound of formula (I) as defined in any one of claims 1 to 7.

9. Process according to claim 8, said process comprising a step of converting a cyanoacetate of formula (II) into a compound of formula (I) :

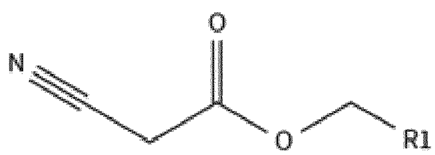

(II)

wherein R1 is as defined in any one of claims 1 to 7.

**10.** Process according to any one of claims 8 or 9, said process comprising :

- a step S1 comprising the reaction of condensation of a compound of formula (II) :

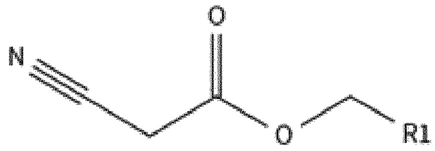
(II),

with a formaldehyde derivative; then
- an optional step S2 comprising the washing of the product obtained in step S1;
- a step S3 comprising the depolymerization of the product obtained at step S1 or S2 to provide a compound of formula (I),
- an optional step S4 of purification of the compound of formula (I) obtained at the end of step S3.

**11.** Process according to any one of claims 8 or 9, said process comprising:

- a step comprising the reaction of compound of formula (II)

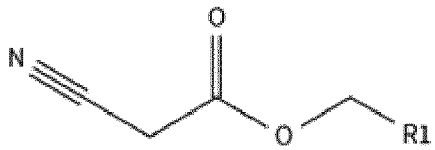
(II),

with a compound of formula (X):

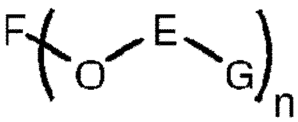
(X)

wherein E is $(CH_2X)_m$, m is comprised between 1 and 20, X is O or S, when n=1, F is selected from the functional groups:

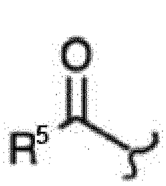
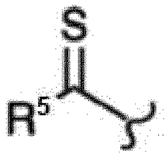
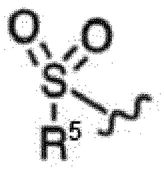
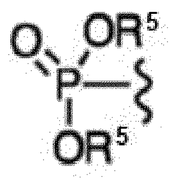

when n=2, F is selected from the functional groups:

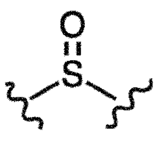
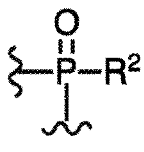
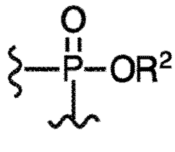

when n= 3, F is:

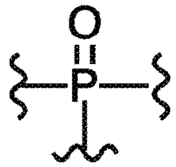

,

G is selected from the functional groups:

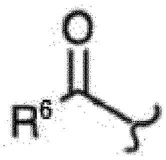

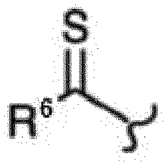

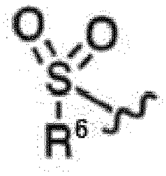

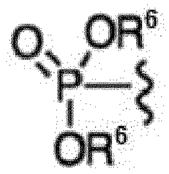

G and F are independently selected from each other, and
R5 and R6 are independently of each other selected from the functional groups H, linear or branched C1-C4 alkyl, C1-C4 halogenated alkyl, carboxy substituted C1-C4 alky, C3-C10 cycloalkyl, aryl, and heterocycloalkyl, or
When n = 1, F may be connected to G through one R7 group selected from $(CH_2)_v$, $(CHR7)_v$, $(CR7R8)_v$, where v ranges from 1 to 6, preferably from 1 to 4, and more preferably v is 1, R7 and R8 being the same or different C1-C4 alkyl groups,
in presence of a catalytic amount of an ammonium or iminium salt (XI);

- an optional step of isolation of the compound obtained (II) obtained.

**12.** Process according to any one of claims 9 to 11, wherein the compound of formula (II) is obtained by one of the following processes P-A, P-B, P-C, P-D or P-E :

- the process P-A of preparation of a compound of formula (II) comprising:

  - contacting a compound of formula (III) with a compound of formula (IV), in the presence of an acid, more preferably under conditions sufficient to yield a compound of formula (II):

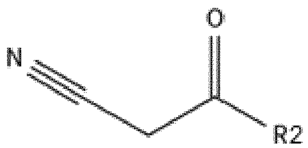

(III)

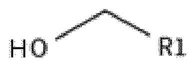

(IV)

  wherein R1 is as defined in any one of claims 1 to 6,
  wherein R2= NRR' with each of R and R' being, independently of each other H, or an alkyl group;
  - optionally separating therefrom the compound of formula (II)

- the process P-B of preparation of a compound of formula (II) comprising:

  - contacting a compound of formula (V) with a compound of formula (IV) under conditions sufficient to yield a compound of formula (II):

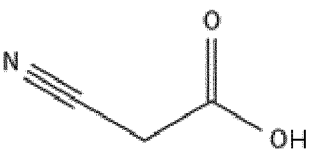

(V)

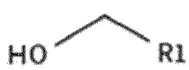

(IV)

wherein R1 is as defined in any one of claims 1 to 7,
- optionally separating therefrom the compound of formula (II)

- the process P-C of preparation of a compound of formula (II) comprising:

- contacting a compound of formula (II') with a compound of formula (IV), under conditions sufficient to yield a compound of formula (II):

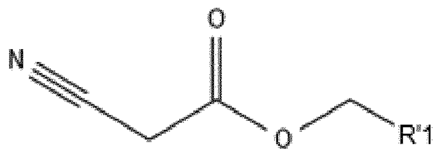 (II') 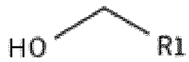 (IV)

wherein R'1 is H or an organic moiety different from R1, and R1 is as defined in any one of claims 1 to 7;
- optionally separating therefrom the compound of formula (II)

- the process P-D of preparation of a compound of formula (II) comprising:

- contacting a compound of formula (VIII) with a compound of formula (IV) under conditions sufficient to yield a compound of formula (II):

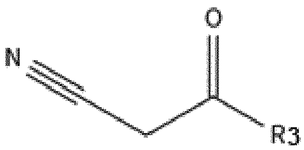 (VIII) 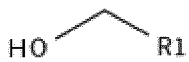 (IV)

wherein R1 is as defined above for formula (I),
wherein R3 is Br, Cl or I;
- optionally separating therefrom the compound of formula (II)

- the process P-E of preparation of a compound of formula (II) comprising:

- contacting a compound of formula (IX) with a compound of formula (IV) under conditions sufficient to yield a compound of formula (II):

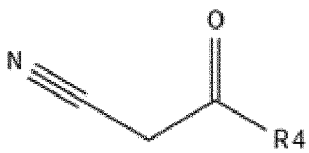 (IX) 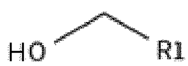 (IV)

wherein R1 is as defined above for formula (I),
wherein R4 is -O-C(=O)-R4, with R4 being selected from alkyl or aryl;
- optionally separating therefrom the compound of formula (II).

**13.** Process according to claim 12, wherein the compound of formula (IV) has a biocarbon content equal to or higher than 20%, preferably equal to or higher than 50%, more preferably equal to or higher than 80%, and even more advantageously equal to or higher than 99%.

**14.** Process according to anyone of claims 12 or 13, wherein the compound of formula (IV) is a compound wherein R1 is selected from alkyl group and heterocycloalkyl, preferably R1 is a C1-C20 alkyl group, more preferably C1-C10 alkyl group, even more preferably C6 alkyl group.

**15.** Process according to anyone of claims 12 to 14, wherein the compound of formula (V) is obtained from a process comprising contacting a compound of formula (VI) with potassium cyanide (KCN) or sodium cyanide (NaCN) under conditions sufficient to yield a compound of formula (V):

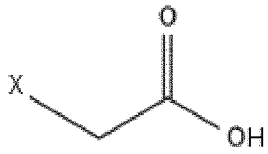
(VI)

wherein X represents a halogen atom, preferably Br or Cl, more preferably Br, and wherein the compound of formula (VI) is preferably obtained from a process comprising contacting a compound of formula (VII) with a halogenating agent H under conditions sufficient to yield a compound of formula (VI):

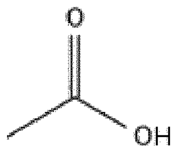
(VII)

**16.** Process according to claim 15, wherein :

- the compound of formula (V) is petroleum-based, or partially biobased or fully biobased (biocarbon content of 100%), and/or
- the compound of formula (VI) is petroleum-based, or partially biobased or even fully biobased (biocarbon content of 100%), and/or
- the compound of formula (VII) is petroleum-based or partially or fully biobased (biocarbon content of 100%).

**17.** Composition, preferably adhesive composition, comprising the compound of formula (I) as defined in anyone of claims 1 to 7, preferably in a content higher than 80%, more preferably equal to or higher than 85%, even more preferably equal to or higher than 90% by weight based on the total weight of the composition

**18.** Composition according to claim 17, having a biocarbon content equal to or higher than 20%, even more preferably higher to or equal to 50%.

**19.** Use of at least one raw material R having a biocarbon content higher than 0%, for the preparation of a compound of formula (I):

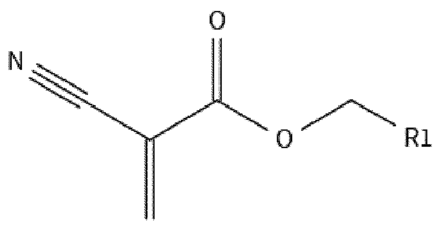
(I)

wherein R1 is H or an organic moiety, said compound of formula (I) having a biocarbon content equal to or higher than 20%.

**20.** Use according to claim 19, wherein the raw material R has a biocarbon content equal to or higher than 41%, preferably equal to or higher than 50%, more preferably equal to or higher than 65%, and even more preferably equal to 100%.

**21.** Use according to any one of claims 19 or 20, wherein the raw material R is selected from the group consisting of: acetic acid, ethanol, n-heptanol, isobutanol, n butanol, 1,10 decanediol, tetrahydrofurfuryl ethanol, preferably it is

ethanol and n-heptanol, even more preferably n-heptanol.

Europäisches Patentamt
European Patent Office
Office européen des brevets

# EUROPEAN SEARCH REPORT

Application Number
EP 22 30 5816

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 2 927 209 A1 (AFINITICA TECHNOLOGIES S L [ES]) 7 October 2015 (2015-10-07)<br>* claims 1-15 *<br>* examples 1-37 *<br>----- | 1-21 | INV.<br>C07C255/23<br>C09J4/00<br>C07D307/00 |
| Y | EP 2 792 372 A1 (INST PHARM & TOXICOLOGY AMMS [CN] ET AL.)<br>22 October 2014 (2014-10-22)<br>* claim 1 *<br>----- | 1-21 | |
| Y | WENDELS SOPHIE ET AL: "Influence of the Macromolecular architecture on the properties of biobased polyurethane tissue adhesives",<br>EUROPEAN POLYMER JOURNAL, PERGAMON PRESS LTD OXFORD, GB,<br>vol. 164, 25 December 2021 (2021-12-25), XP086938330,<br>ISSN: 0014-3057, DOI: 10.1016/J.EURPOLYMJ.2021.110968<br>[retrieved on 2021-12-25]<br>* abstract *<br>* page 2, column 1 *<br>* table 1 *<br>* page 3, column 2, paragraph 3 *<br>----- | 1-21 | TECHNICAL FIELDS SEARCHED (IPC)<br>C07C<br>C07D<br>C09J |
| Y | WO 2013/076626 A1 (SABIC INNOVATIVE PLASTICS IP [NL]) 30 May 2013 (2013-05-30)<br>* paragraph [0002] - paragraph [0005] *<br>* paragraph [0053] *<br>----- | 1-21 | |
| Y | EP 3 645 268 B1 (3M INNOVATIVE PROPERTIES CO [US]) 26 May 2021 (2021-05-26)<br>* paragraph [0010] *<br>* paragraph [0019] - paragraph [0020] *<br>----- | 1-21 | |

The present search report has been drawn up for all claims

1

| Place of search | Date of completion of the search | Examiner |
|---|---|---|
| The Hague | 10 November 2022 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 22 30 5816

This annex lists the patent family members relating to the patent documents cited in the above-mentioned European search report. The members are as contained in the European Patent Office EDP file on
The European Patent Office is in no way liable for these particulars which are merely given for the purpose of information.

10-11-2022

| Patent document cited in search report | Publication date | Patent family member(s) | Publication date |
|---|---|---|---|
| EP 2927209 A1 | 07-10-2015 | CN 106470968 A | 01-03-2017 |
| | | EP 2927209 A1 | 07-10-2015 |
| | | EP 3126321 A1 | 08-02-2017 |
| | | JP 6452262 B2 | 16-01-2019 |
| | | JP 2017514796 A | 08-06-2017 |
| | | TW 201536718 A | 01-10-2015 |
| | | US 2017022151 A1 | 26-01-2017 |
| | | WO 2015150882 A1 | 08-10-2015 |
| EP 2792372 A1 | 22-10-2014 | CN 103083718 A | 08-05-2013 |
| | | EP 2792372 A1 | 22-10-2014 |
| | | JP 6334405 B2 | 30-05-2018 |
| | | JP 2014532488 A | 08-12-2014 |
| | | US 2014369952 A1 | 18-12-2014 |
| | | WO 2013064059 A1 | 10-05-2013 |
| WO 2013076626 A1 | 30-05-2013 | CN 104066791 A | 24-09-2014 |
| | | EP 2782962 A1 | 01-10-2014 |
| | | US 2013137804 A1 | 30-05-2013 |
| | | WO 2013076626 A1 | 30-05-2013 |
| EP 3645268 B1 | 26-05-2021 | EP 3645268 A1 | 06-05-2020 |
| | | WO 2019003158 A1 | 03-01-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6977278 B
- WO 2015150882 A
- US 3927078 A
- US 3219630 A
- US 3931412 A
- US 4100200 A
- WO 9640695 A
- CN 201510810110
- CN 201610662675
- WO 2017021785 A1
- US 5824180 A
- US 6503959 B
- WO 2016038514 A


### Non-patent literature cited in the description


- Adhesives Technology Handbook. William Andrew, 2008
- Ullmann's Encyclopedia of Industrial Chemistry. Wiley-VCH, 2000
- **WANG.** *Chem Sci.,* 2021, vol. 12, 10259-10265
- **ANDRUSSOW L.** The catalytic oxydation of ammonia-methane-mixtures to hydrogen cyanide. *Angewandte Chemie,* 1935, vol. 48 (37), 593-595
- **PRADYOT PATNAIK.** Handbook of Inorganic Chemicals. McGraw-Hill, 2002
- *Chemistry, a European journal,* vol. 24 (71), 18903-18906
- *Asian Journal of Chemistry,* 2018, vol. 30 (10), 2310-2316
- *CHEMICAL ABSTRACTS,* 64-19-7
- *Zhanjie Zazhishe,* 2014, vol. 35 (2), 67-68
- *CHEMICAL ABSTRACTS,* 27176-87-0
- **T.M. WERKHOVEN et al.** *Eur. J. Org. Chem.,* 1999, 2909-2914
- **J. KREMSER et al.** *Chem. Comm.,* 2017, vol. 53 (96), 12938-12941